# EUROPEAN PATENT SPECIFICATION

(11) **EP 1 226 244 B1**
(45) Date of publication and mention of the grant of the patent: **28.07.2004**
(21) Application number: 00972995.5
(22) Date of filing: 01.11.2000
(51) Int. Cl.: C12N 15/12, C12N 15/62, C07K 14/705, A61K 38/17

(54) **POLYPEPTIDES WITH NON-NATURAL PRIMARY SIGNALLING MOTIFS**
POLYPEPTIDE DIE UNNATÜRLICHE SIGNALMOTIVE ENTHALTEN
POLYPEPTIDES A MOTIFS DE SIGNALISATION PRIMAIRE NON NATURELLE

(30) Priority: 01.11.1999 GB 9925848
(43) Date of publication of application: 31.07.2002
(73) Proprietor: Celltech R&D Limited, Slough, Berkshire SL1 3WE (GB)
(72) Inventor: FINNEY, Helene Margaret, Maidenhead, Berkshire SL6 4DQ (GB); LAWSON, Alastair David Griffiths, Alresford, Hampshire SO2 0NX (GB)
(74) Representative: Mercer, Christopher Paul
(86) International application number: PCT/GB2000/004183
(87) International publication number: WO 2001/032709

(56) References cited:
- WO-A-00/63372
- WO-A-00/63374
- WO-A-94/17095
- WO-A-97/23613
- WO-A-99/00494
- BONNEROT C ET AL.: "Syk protein tyrosine kinase regulates Fc receptor gamma-chain-mediated transport to lysosomes" EMBO JOURNAL, vol. 17, no. 16, 17 August 1998 (1998-08-17), pages 4606-4616, XP002166682
- FITZER-ATTAS C ET AL.: "Tyrosine kinase chimeras for antigen-selective T-body therapy" ADVANCED DRUG DELIVERY REVIEWS, vol. 31, no. 1-2, 6 April 1998 (1998-04-06), pages 171-182, XP000993389
- ISAKOV N: "Role of immunoreceptor tyrosine-based activation motif in signal transduction from antigen and Fc receptors" ADVANCES IN IMMUNOLOGY, vol. 69, 1998, pages 183-247, XP000987100

### SEQUENCE LISTING

<110> CELLTECH CHIROSCIENCE LIMITED
<120> POLYPEPTIDES WITH NON-NATURAL PRIMARY SIGNALLING MOTIFS
<130> P025965WO
<140> PCT/GB00/04183
   <141> 2000-11-01
<150> GB9925848.5
   <151> 1999-11-01
<160> 82
<170> SeqWin99, version 1.02
<210> 1
   <211> 30
   <212> PRT
   <213> Homo sapiens
<400> 1
   Gly Gln Asn Gln Leu Tyr Asn Glu Leu Asn Leu Gly Arg Arg Glu Glu
   1               5                   10                  15
   Tyr Asp Val Leu Asp Lys Arg Arg Gly Arg Asp Pro Glu Met
               20                  25                  30
<210> 2
   <211> 30
   <212> PRT
   <213> Homo sapiens
<400> 2
   Arg Lys Asn Pro Gln Glu Gly Leu Tyr Asn Glu Leu Gln Lys Asp Lys
   1               5                   10                  15
   Met Ala Glu Ala Tyr Ser Glu Ile Gly Met Lys Gly Glu Arg
               20                  25                  30
<210> 3
   <211> 27
   <212> PRT
   <213> Homo sapiens
<400> 3
   Arg Gly Lys Gly His Asp Gly Leu Tyr Gln Gly Leu Ser Thr Ala Thr
   1               5                   10                  15
   Lys Asp Thr Tyr Asp Ala Leu His Met Gln Ala
               20                  25
<210> 4
   <211> 27
   <212> PRT
   <213> Homo sapiens
<400> 4
   Tyr Glu Lys Ser Asp Gly Val Tyr Thr Gly Leu Ser Thr Arg Asn Gln
   1 5 10 15
   Glu Thr Tyr Glu Thr Leu Lys His Glu Lys Pro
   20 25
<210> 5
   <211> 31
   <212> PRT
   <213> Homo sapiens
<400> 5
   Gly Asn Lys Asx Pro Glu Asp Arg Val Tyr Glu Glu Leu Asn Ile Tyr
   1 5 10 15
   Ser Ala Thr Tyr Ser Glu Leu Glu Asp Pro Gly Glu Met Ser Pro
   20 25 30
<210> 6
   <211> 31
   <212> PRT
   <213> Homo sapiens
<400> 6
   Lys Gln Thr Leu Leu Pro Asn Asp Gln Leu Tyr Gln Pro Leu Lys Asp
   1 5 10 15
   Arg Glu Asp Asp Gln Tyr Ser His Leu Gln Gly Asn Gln Leu Arg
   20 25 30
<210> 7
   <211> 31
   <212> PRT
   <213> Homo sapiens
<400> 7
   Ala Leu Leu Arg Asn Asp Gln Val Tyr Gln Pro Leu Arg Asp Arg Asp
   1 5 10 15
   Asp Ala Gln Tyr Ser His Leu Gly Gly Asn Trp Ala Arg Asn Lys
   20 25 30
<210> 8
   <211> 33
   <212> PRT
   <213> Homo sapiens
<400> 8
   Gln Asn Lys Glu Arg Pro Pro Pro Val Pro Asn Pro Asp Tyr Glu Pro
   1 5 10 15
   Ile Arg Lys Gly Gln Arg Asp Leu Tyr Ser Gly Leu Asn Gln Arg Arg
   20 25 30
   Ile
<210> 9
   <211> 28
   <212> PRT
   <213> Homo sapiens
<400> 9
   His Val Asp Asn Glu Tyr Ser Gln Pro Pro Arg Asn Ser Arg Leu Ser
   1 5 10 15
   Ala Tyr Pro Ala Leu Glu Gly Val Leu His Arg Ser
   20 25
<210> 10
   <211> 33
   <212> PRT
   <213> Homo sapiens
<400> 10
   Pro Pro Arg Thr Cys Asp Asp Thr Val Thr Tyr Ser Ala Leu His Lys
   1 5 10 15
   Arg Gln Val Gly Asp Tyr Glu Asn Val Ile Pro Asp Phe Pro Glu Asp
   20 25 30
   Glu
<210> 11
   <211> 33
   <212> PRT
   <213> Homo sapiens
<400> 11
   Glu Tyr Glu Asp Glu Asn Leu Tyr Glu Gly Leu Asn Leu Asp Asp Cys
   1 5 10 15
   Ser Met Tyr Glu Asp Ile Ser Arg Gly Leu Gln Gly Thr Tyr Gln Asp
   20 25 30
   Val
<210> 12
   <211> 32
   <212> PRT
   <213> Homo sapiens
<400> 12
   Lys Ala Gly Met Glu Glu Asp His Thr Tyr Glu Gly Leu Asp Ile Asp
   1 5 10 15
   Gln Thr Ala Thr Tyr Glu Asp Ile Val Thr Leu Arg Thr Gly Glu Val
   20 25 30
<210> 13
   <211> 33
   <212> PRT
   <213> Homo sapiens
<400> 13
   Pro Leu Pro Asn Pro Arg Thr Ala Ala Ser Ile Tyr Glu Glu Leu Leu
   1 5 10 15
   Lys His Asp Thr Asn Ile Tyr Cys Arg Met Asp His Lys Ala Glu Val
   20 25 30
   Ala
<210> 14
   <211> 27
   <212> PRT
   <213> Homo sapiens
<400> 14 <210> 15
   <211> 26
   <212> PRT
   <213> Artificial Sequence
<220>
   <223> Non-natural primary signalling motif
<400> 15
<210> 16
   <211> 27
   <212> PRT
   <213> Artificial Sequence
<220>
   <223> Non-natural primary signalling motif
<400> 16
   Gly Gln Asp Gly Leu Tyr Gln Glu Leu Asn Thr Arg Ser Arg Asp Glu
   1               5                   10                  15
   Ala Tyr Ser Val Leu Glu Gly Arg Lys Ala Arg
               20                  25
<210> 17
   <211> 200
   <212> DNA
   <213> Homo sapiens
<400> 17
   cgactagtga caaaactcac acatgcccac cgtgcccaaa agggaaacac ctttgtccaa   60
   gtcccctatt tcccggacct tctaagcccg gcgccttttg ggtgctggtg gtggttggtg   120
   gagtcctggc ttgctatagc ttgctagtaa cagtggcctt tattattttc tgggtgacgc   180
   gtggatcctg agaattcata                                               200
<210> 18
   <211> 65
   <212> DNA
   <213> Artificial Sequence
<220>
<223> oligonucleotide for chimeric receptor construction
<400> 18
   cgactagtga caaaactcac acatgcccac cgtgcccaaa agggaaacac ctttgtccaa   60
   ctccc                                                               65
<210> 19
   <211> 63
   <212> DNA
   <213> Artificial Sequence
<220>
   <223> oligonucleotide for chimeric receptor construction
<400> 19
   gccttttggg tgctggtggt ggttggtgga gtcctggctt gctatagctt gctagtaaca   60
   gtg                                                                 63
<210> 20
   <211> 67
   <212> DNA
   <213> Artificial Sequence
<220>
   <223> oligonucleotide for chimeric receptor construction
<400> 20
   tatgaattct caggatccac gcgtcaccca gaaaataata aaggccactg ttactagcaa    60
   gctatag                                                              67
<210> 21
   <211> 59
   <212> DNA
   <213> Artificial Sequence
<220>
   <223> oligonucleotide for chimeric receptor construction
<400> 21
   caccaccagc acccaaaagg cgccgggctt agaaggtccg ggaaataggg gacttggac     59
<210> 22
   <211> 86
   <212> DNA
   <213> Artificial Sequence
<220>
   <223> oligonucleotide for chimeric receptor construction
<400> 22
   gatcctggtt tctcatgctt cagagtctcg taagtctcct ggttcctggt gctcaggccc    60
   gtgtaacacc atctgatttc tcatat                                         86
<210> 23
   <211> 86
   <212> DNA
   <213> Artificial Sequence
<220>
<223> oligonucleotide for chimeric receptor construction
<400> 23
   gatcctggtt tctcatgctt cagagtatcg taagtctcct ggttcctggt gctcaggccc    60
   gtgtaacacc atctgatttc tcatat                                         86
<210> 24
   <211> 87
   <212> DNA
   <213> Artificial Sequence
<220>
   <223> oligonucleotide for chimeric receptor construction
<400> 24
   gatcatatga gaaatcagat ggtgtttaca cgggcctgag caccaggaac caggagactt   60
   acgagactct gaagcatgag aaaccag                                       87
<210> 25
   <211> 87
   <212> DNA
   <213> Artificial Sequence
<220>
   <223> oligonucleotide for chimeric receptor construction
<400> 25
   gatcatatga gaaatcagat ggtgtttaca cgggcctgag caccaggaac caggagactt   60
   acgatactct gaagcatgag aaaccag                                       87
<210> 26
   <211> 87
   <212> DNA
   <213> Artificial Sequence
<220>
   <223> oligonucleotide for chimeric receptor construction
<400> 26
   gatccggcct gcatgtgaag ggcgtcgtag gtgtccttgg tggctgtact gagaccctgg   60
   taaaggccat cgtgcccctg tcccctt                                       87
<210> 27
   <211> 87
   <212> DNA
   <213> Artificial Sequence
<220>
   <223> oligonucleotide for chimeric receptor construction
<400> 27
   gatcaagggg caaggggcac gatggccttt accagggtct cagtacagcc accaaggaca   60
   cctacgacgc ccttcacatg caggccg                                       87
<210> 28
   <211> 96
   <212> DNA
   <213> Artificial Sequence
<220>
   <223> oligonucleotide for chimeric receptor construction
<400> 28
   gatccgcgct cgcctttcat cccaatctca ctgtaggcct ccgccatctt atctttctgc   60
   agttcattgt acaggccttc ctgagggttc ttcctt                             96
<210> 29
   <211> 96
   <212> DNA
   <213> Artificial Sequence
<220>
   <223> oligonucleotide for chimeric receptor construction
<400> 29
   gatcaaggaa gaaccctcag gaaggcctgt acaatgaact gcagaaagat aagatggcgg   60
   aggcctacag tgagattggg atgaaaggcg agcgcg                             96
<210> 30
   <211> 96
   <212> DNA
   <213> Artificial Sequence
<220>
   <223> oligonucleotide for chimeric receptor construction
<400> 30
   gatcccatct cagggtcccg gccacgtctc ttgtccaaaa catcgtactc ctctcttcgt   60
   cctagattga gctcgttata gagctggttc tggcct                             96
<210> 31
   <211> 96
   <212> DNA
   <213> Artificial Sequence
<220>
   <223> oligonucleotide for chimeric receptor construction
<400> 31
   gatcaggcca gaaccagctc tataacgagc tcaatctagg acgaagagag gagtacgatg   60
   ttttggacaa gagacgtggc cgggaccctg agatgg                             96
<210> 32
   <211> 99
   <212> DNA
   <213> Artificial Sequence
<220>
   <223> oligonucleotide for chimeric receptor construction
<400> 32
   gatcaggaaa caaggttcca gaggatcgtg tttatgaaga attaaacata tattcagcta   60
   cttacagtga gttggaagac ccaggggaaa tgtctcctg                          99
<210> 33
   <211> 99
   <212> DNA
   <213> Artificial Sequence
<220>
   <223> oligonucleotide for chimeric receptor construction
<400> 33
   gatccaggag acatttcccc tgggtcttcc aactcactgt aagtagctga atatatgttt 60
   aattcttcat aaacacgatc ctctggaacc ttgtttcct 99
<210> 34
   <211> 99
   <212> DNA
   <213> Artificial Sequence
<220>
   <223> oligonucleotide for chimeric receptor construction
<400> 34
   gatcaaagca gactctgttg cccaatgacc agctctacca gcccctcaag gatcgagaag 60
   atgaccagta cagccacctt caaggaaacc agttgaggg 99
<210> 35
   <211> 99
   <212> DNA
   <213> Artificial Sequence
<220>
   <223> oligonucleotide for chimeric receptor construction
<400> 35
   gatcccctca actggtttcc ttgaaggtgg ctgtactggt catcttctcg atccttgagg 60
   ggctggtaga gctggtcatt gggcaacaga gtctgcttt 99
<210> 36
   <211> 99
   <212> DNA
   <213> Artificial Sequence
<220>
   <223> oligonucleotide for chimeric receptor construction
<400> 36
   gatcagctct gttgaggaat gaccaggtct atcagcccct ccgagatcga gatgatgctc 60
   agtacagcca ccttggagga aactgggctc ggaacaagg 99
<210> 37
   <211> 99
   <212> DNA
   <213> Artificial Sequence
<220>
   <223> oligonucleotide for chimeric receptor construction
<400> 37
   gatcccttgt tccgagccca gtttcctcca aggtggctgt actgagcatc atctcgatct   60
   cggaggggct gatagacctg gtcattcctc aacagagct                          99
<210> 38
   <211> 105
   <212> DNA
   <213> Artificial Sequence
<220>
   <223> oligonucleotide for chimeric receptor construction
<400> 38
   gatcacaaaa caaggagagg ccaccacctg ttcccaaccc agactatgag cccatccgga   60
   aaggccagcg ggacctgtat tctggcctga atcagagacg catcg                  105
<210> 39
   <211> 105
   <212> DNA
   <213> Artificial Sequence
<220>
   <223> oligonucleotide for chimeric receptor construction
<400> 39
   gatccgatgc gtctctgatt caggccagaa tacaggtccc gctggccttt ccggatgggc   60
   tcatagtctg ggttgggaac aggtggtggc ctctccttgt tttgt                  105
<210> 40
   <211> 90
   <212> DNA
   <213> Artificial Sequence
<220>
   <223> oligonucleotide for chimeric receptor construction
<400> 40
   gatcacacgt ggataacgaa tacagccaac ctcccaggaa ctcccgcctg tcagcttatc   60
   cagctctgga aggggttctg catcgctccg                                    90
<210> 41
   <211> 90
   <212> DNA
   <213> Artificial Sequence
<220>
   <223> oligonucleotide for chimeric receptor construction
<400> 41
   gatccggagc gatgcagaac cccttccaga gctggataag ctgacaggcg ggagttcctg 60
   ggaggttggc tgtattcgtt atccacgtgt 90
<210> 42
   <211> 105
   <212> DNA
   <213> Artificial Sequence
<220>
   <223> oligonucleotide for chimeric receptor construction
<400> 42
   gatcacctcc ccggacctgc gatgacacgg tcacttattc agcattgcac aagcgccaag 60
   tgggcgacta tgagaacgtc attccagatt ttccagaaga tgagg 105
<210> 43
   <211> 105
   <212> DNA
   <213> Artificial Sequence
<220>
   <223> oligonucleotide for chimeric receptor construction
<400> 43
   gatccctcat cttctggaaa atctggaatg acgttctcat agtcgcccac ttggcgcttg 60
   tgcaatgctg aataagtgac cgtgtcatcg caggtccggg gaggt 105
<210> 44
   <211> 105
   <212> DNA
   <213> Artificial Sequence
<220>
   <223> oligonucleotide for chimeric receptor construction
<400> 44
   gatcagaata tgaagatgaa aacctttatg aaggcctgaa cctggacgac tgctccatgt 60
   atgaggacat ctcccggggc ctccagggca cctaccagga tgtgg 105
<210> 45
   <211> 105
   <212> DNA
   <213> Artificial Sequence
<220>
   <223> oligonucleotide for chimeric receptor construction
<400> 45
   gatcccacat cctggtaggt gccctggagg ccccgggaga tgtcctcata catggagcag   60
   tcgtccaggt tcaggccttc ataaaggttt tcatcttcat attct                  105
<210> 46
   <211> 102
   <212> DNA
   <213> Artificial Sequence
<220>
   <223> oligonucleotide for chimeric receptor construction
<400> 46
   gatcaaaggc tggcatggag gaagatcaca cctacgaggg cctggacatt gaccagacag   60
   ccacctatga ggacatagtg acgctgcgga caggggaagt gg                     102
<210> 47
   <211> 102
   <212> DNA
   <213> Artificial Sequence
<220>
   <223> oligonucleotide for chimeric receptor construction
<400> 47
   gatcccactt cccctgtccg cagcgtcact atgtcctcat aggtggctgt ctggtcaatg   60
   tccaggccct cgtaggtgtg atcttcctcc atgccagcct tt                     102
<210> 48
   <211> 105
   <212> DNA
   <213> Artificial Sequence
<220>
   <223> oligonucleotide for chimeric receptor construction
<400> 48
   gatcacccct acccaacccc aggacagcag cttccatcta tgaggaattg ctaaaacatg   60
   acacaaacat ttactgccgg atggaccaca aagcagaagt ggctg                  105
<210> 49
   <211> 105
   <212> DNA
   <213> Artificial Sequence
<220>
   <223> oligonucleotide for chimeric receptor construction
<400> 49
   gatccagcca cttctgcttt gtggtccatc cggcagtaaa tgtttgtgtc atgttttagc   60
   aattcctcat agatggaagc tgctgtcctg gggttgggta ggggt                   105
<210> 50
   <211> 102
   <212> DNA
   <213> Artificial Sequence
<220>
   <223> oligonucleotide for chimeric receptor construction
<400> 50
   gatcaaggct cctgcacagt gactacatga acatgactcc tcgccgacca gggccaaccc   60
   gcaagcatta ccagccctat gccccaccac gcgacttcgc ag                      102
<210> 51
   <211> 102
   <212> DNA
   <213> Artificial Sequence
<220>
   <223> oligonucleotide for chimeric receptor construction
<400> 51
   gatcctgcga agtcgcgtgg tggggcatag ggctggtaat gcttgcgggt tggccctggt   60
   cggcgaggag tcatgttcat gtagtcactg tgcaggagcc tt                      102
<210> 52
   <211> 72
   <212> DNA
   <213> Artificial Sequence
<220>
   <223> oligonucleotide for chimeric receptor construction
<400> 52
   gatcaatgat cgaaacatac aaccaaactt ctccccgatc tgcggccact ggactgccca   60
   tcagcatgaa ag                                                        72
<210> 53
   <211> 72
   <212> DNA
   <213> Artificial Sequence
<220>
   <223> oligonucleotide for chimeric receptor construction
<400> 53
   gatcctttca tgctgatggg cagtccagtg gccgcagatc ggggagaagt ttggttgtat   60
   gtttcgatca tt                                                        72
<210> 54
   <211> 87
   <212> DNA
   <213> Artificial Sequence
<220>
   <223> oligonucleotide for chimeric receptor construction
<400> 54
   gatcaggaca ggacggtcta taccaggagc tgaacaccag gtcgcgggat gaggcctact   60
   cggtcctaga aggccgcaag gcaagag                                       87
<210> 55
   <211> 84
   <212> DNA
   <213> Artificial Sequence
<220>
   <223> oligonucleotide for chimeric receptor construction
<400> 55
   gatcaggaca ggacggtcta taccaggagc tgaacaccag gtcgcgggat gagtactcgg   60
   tcctagaagg ccgcaaggca agag                                          84
<210> 56
   <211> 84
   <212> DNA
   <213> Artificial Sequence
<220>
   <223> oligonucleotide for chimeric receptor construction
<400> 56
   gatcctcttg ccttgcggcc ttctaggacc gagtactcat cccgcgacct ggtgttcagc   60
   tcctggtata gaccgtcctg tcct                                          84
<210> 57
   <211> 87
   <212> DNA
   <213> Artificial Sequence
<220>
   <223> oligonucleotide for chimeric receptor construction
<400> 57
   gatcctcttg ccttgcggcc ttctaggacc gagtaggcct catcccgcga cctggtgttc 60
   agctcctggt atagaccgtc ctgtcct 87
<210> 58
   <211> 117
   <212> DNA
   <213> Artificial Sequence
<220>
   <223> oligonucleotide for chimeric receptor construction
<400> 58
   gatcacggag ggaccagagg ctgccccccg atgcccacaa gccccctggg ggaggcagtt 60
   tccggacccc catccaagag gagcaggcgg acgcccactc caccctggcc aagatcg 117
<210> 59
   <211> 117
   <212> DNA
   <213> Artificial Sequence
<220>
   <223> oligonucleotide for chimeric receptor construction
<400> 59
   gatccgatct tggccagggt ggagtgggcg tcggcctgct cctcttggat gggggtccgg 60
   aaactgcctc ccccaggggg cttttgggca tcggggggca gcctctggtc cctccgt 117
<210> 60
   <211> 34
   <212> PRT
   <213> Artificial Sequence
<220>
   <223> sequence employed in the construction of chimeric receptors <400> 60
<210> 61
   <211> 34
   <212> PRT
   <213> Artificial Sequence
<220>
   <223> sequence employed in the construction of chimeric receptors
<400> 61
   Gly Ser Arg Lys Asn Pro Gln Glu Gly Leu Tyr Asn Glu Leu Gln Lys
   1 5 10 15
   Asp Lys Met Ala Glu Ala Tyr Ser Glu Ile Gly Met Lys Gly Glu Arg
   20 25 30
   Gly Ser
<210> 62
   <211> 31
   <212> PRT
   <213> Artificial Sequence
<220>
   <223> sequence employed in the construction of chimeric receptors
<400> 62
   Gly Ser Arg Gly Lys Gly His Asp Gly Leu Tyr Gln Gly Leu Ser Thr
   1 5 10 15
   Ala Thr Lys Asp Thr Tyr Asp Ala Leu His Met Gln Ala Gly Ser
   20 25 30
<210> 63
   <211> 31
   <212> PRT
   <213> Artificial Sequence
<220>
   <223> sequence employed in the construction of chimeric receptors
<400> 63
   Gly Ser Tyr Glu Lys Ser Asp Gly Val Tyr Thr Gly Leu Ser Thr Arg
   1 5 10 15
   Asn Gln Glu Thr Tyr Glu Thr Leu Lys His Glu Lys Pro Gly Ser
   20 25 30
<210> 64
   <211> 31
   <212> PRT
   <213> Artificial Sequence
<220>
   <223> sequence employed in the construction of chimeric receptors
<400> 64
   Gly Ser Tyr Glu Lys Ser Asp Gly Val Tyr Thr Gly Leu Ser Thr Arg
   1 5 10 15
   Asn Gln Glu Thr Tyr Asp Thr Leu Lys His Glu Lys Pro Gly Ser
   20 25 30
<210> 65
   <211> 35
   <212> PRT
   <213> Artificial Sequence
<220>
   <223> sequence employed in the construction of chimeric receptors
<400> 65
   Gly Ser Gly Asn Lys Asx Pro Glu Asp Arg Val Tyr Glu Glu Leu Asn
   1               5                   10                  15
   Ile Tyr Ser Ala Thr Tyr Ser Glu Leu Glu Asp Pro Gly Glu Met Ser
               20                  25                  30
   Pro Gly Ser
           35
<210> 66
   <211> 35
   <212> PRT
   <213> Artificial Sequence
<220>
   <223> sequence employed in the construction of chimeric receptors
<400> 66
   Gly Ser Lys Gln Thr Leu Leu Pro Asn Asp Gln Leu Tyr Gln Pro Leu
   1               5                   10                  15
   Lys Asp Arg Glu Asp Asp Gln Tyr Ser His Leu Gln Gly Asn Gln Leu
               20                  25                  30
   Arg Gly Ser
           35
<210> 67
   <211> 35
   <212> PRT
   <213> Artificial Sequence
<220>
   <223> sequence employed in the construction of chimeric receptors
<400> 67
   Gly Ser Ala Leu Leu Arg Asn Asp Gln Val Tyr Gln Pro Leu Arg Asp
   1 5 10 15
   Arg Asp Asp Ala Gln Tyr Ser His Leu Gly Gly Asn Trp Ala Arg Asn
   20 25 30
   Lys Gly Ser
   35
<210> 68
   <211> 37
   <212> PRT
   <213> Artificial Sequence
<220>
   <223> sequence employed in the construction of chimeric receptors
<400> 68
   Gly Ser Gln Asn Lys Glu Arg Pro Pro Pro Val Pro Asn Pro Asp Tyr
   1 5 10 15
   Glu Pro Ile Arg Lys Gly Gln Arg Asp Leu Tyr Ser Gly Leu Asn Gln
   20 25 30
   Arg Arg Ile Gly Ser
   35
<210> 69
   <211> 32
   <212> PRT
   <213> Artificial Sequence
<220>
   <223> sequence employed in the construction of chimeric receptors
<400> 69
   Gly Ser His Val Asp Asn Glu Tyr Ser Gln Pro Pro Arg Asn Ser Arg
   1 5 10 15
   Leu Ser Ala Tyr Pro Ala Leu Glu Gly Val Leu His Arg Ser Gly Ser
   20 25 30
<210> 70
   <211> 37
   <212> PRT
   <213> Artificial Sequence
<220>
   <223> sequence employed in the construction of chimeric receptors
<400> 70
   Gly Ser Pro Pro Arg Thr Cys Asp Asp Thr Val Thr Tyr Ser Ala Leu
   1 5 10 15
   His Lys Arg Gln Val Gly Asp Tyr Glu Asn Val Ile Pro Asp Phe Pro
   20 25 30
   Glu Asp Glu Gly Ser
   35
<210> 71
   <211> 37
   <212> PRT
   <213> Artificial Sequence
<220>
   <223> sequence employed in the construction of chimeric receptors
<400> 71
   Gly Ser Glu Tyr Glu Asp Glu Asn Leu Tyr Glu Gly Leu Asn Leu Asp
   1 5 10 15
   Asp Cys Ser Met Tyr Glu Asp Ile Ser Arg Gly Leu Gln Gly Thr Tyr
   20 25 30
   Gln Asp Val Gly Ser
   35
<210> 72
   <211> 36
   <212> PRT
   <213> Artificial Sequence
<220>
   <223> sequence employed in the construction of chimeric receptors
<400> 72
   Gly Ser Lys Ala Gly Met Glu Glu Asp His Thr Tyr Glu Gly Leu Asp
   1 5 10 15
   Ile Asp Gln Thr Ala Thr Tyr Glu Asp Ile Val Thr Leu Arg Thr Gly
   20 25 30
   Glu Val Gly Ser
   35
<210> 73
   <211> 37
   <212> PRT
   <213> Artificial Sequence
<220>
   <223> sequence employed in the construction of chimeric receptors
<400> 73
   Gly Ser Pro Leu Pro Asn Pro Arg Thr Ala Ala Ser Ile Tyr Glu Glu
    1               5                   10                  15
   Leu Leu Lys His Asp Thr Asn Ile Tyr Cys Arg Met Asp His Lys Ala
               20                  25                  30
   Glu Val Ala Gly Ser
           35
<210> 74
   <211> 30
   <212> PRT
   <213> Artificial Sequence
<220>
   <223> sequence employed in the construction of chimeric receptors
<400> 74
   Gly Ser Gly Gln Asp Gly Leu Tyr Gln Glu Leu Asn Thr Arg Ser Arg
   1               5                   10                  15
   Asp Glu Tyr Ser Val Leu Glu Gly Arg Lys Ala Arg Gly Ser
               20                  25                  30
<210> 75
   <211> 31
   <212> PRT
   <213> Artificial Sequence
<220>
   <223> sequence employed in the construction of chimeric receptors
<400> 75
   Gly Ser Gly Gln Asp Gly Leu Tyr Gln Glu Leu Asn Thr Arg Ser Arg
   1               5                   10                  15
   Asp Glu Ala Tyr Ser Val Leu Glu Gly Arg Lys Ala Arg Gly Ser
               20                  25                  30
<210> 76
   <211> 36
   <212> PRT
   <213> Artificial Sequence
<220>
   <223> sequence employed in the construction of chimeric receptors
<400> 76
   Gly Ser Arg Leu Leu His Ser Asp Tyr Met Asn Met Thr Pro Arg Arg
   1 5 10 15
   Pro Gly Pro Thr Arg Lys His Tyr Gln Pro Tyr Ala Pro Pro Arg Asp
   20 25 30
   Phe Ala Gly Ser
   35
<210> 77
   <211> 26
   <212> PRT
   <213> Artificial Sequence
<220>
   <223> sequence employed in the construction of chimeric receptors
<400> 77
   Gly Ser Met Ile Glu Thr Tyr Asn Gln Thr Ser Pro Arg Ser Ala Ala
   1 5 10 15
   Thr Gly Leu Pro Ile Ser Met Lys Gly Ser
   20 25
<210> 78
   <211> 41
   <212> PRT
   <213> Artificial Sequence
<220>
   <223> sequence employed in the construction of chimeric receptors
<400> 78
   Gly Ser Arg Arg Asp Gln Arg Leu Pro Pro Asp Ala His Lys Pro Pro
   1 5 10 15
   Gly Gly Gly Ser Phe Arg Thr Pro Ile Gln Glu Glu Gln Ala Asp Ala
   20 25 30
   His Ser Thr Leu Ala Lys Ile Gly Ser
   35 40
<210> 79
   <211> 32
   <212> PRT
   <213> Homo sapiens
<400> 79
   Arg Leu Leu His Ser Asp Tyr Met Asn Met Thr Pro Arg Arg Pro Gly
   1               5                   10                  15
   Pro Thr Arg Lys His Tyr Gln Pro Tyr Ala Pro Pro Arg Asp Phe Ala
               20                  25                  30
<210> 80
   <211> 22
   <212> PRT
   <213> Homo sapiens
<400> 80
   Met Ile Glu Thr Tyr Asn Gln Thr Ser Pro Arg Ser Ala Ala Thr Gly
   1               5                   10                  15
   Leu Pro Ile Ser Met Lys
               20
<210> 81
   <211> 37
   <212> PRT
   <213> Homo sapiens
<400> 81
   Arg Arg Asp Gln Arg Leu Pro Pro Asp Ala His Lys Pro Pro Gly Gly
   1               5                   10                  15
   Gly Ser Phe Arg Thr Pro Ile Gln Glu Glu Gln Ala Asp Ala His Ser
               20                  25                  30
   Thr Leu Ala Lys Ile
           35
<210> 82
   <211> 62
   <212> PRT
   <213> Homo sapiens
<400> 82
   Thr Ser Asp Lys Thr His Thr Cys Pro Pro Cys Pro Lys Gly Lys His
   1               5                   10                  15
   Leu Cys Pro Ser Pro Leu Phe Pro Gly Pro Ser Lys Pro Gly Ala Phe
               20                  25                  30
   Trp Val Leu Val Val Val Gly Gly Val Leu Ala Cys Tyr Ser Leu Leu
           35                  40                  45
   Val Thr Val Ala Phe Ile Ile Phe Trp Val Thr Arg Gly Ser
       50                  55                  60

## Description

The present invention relates to novel cytoplasmic signalling molecules, the nucleic acids that encode them, and the use of such polypeptides and nucleic acids in medicine and research.

Throughout this application various publications are reference and year of publication. Full citations for these publications are provided following the detailed description of the invention and examples.

Research in the area of immune cell signalling has yielded a considerable amount of information about the signal transduction events that occur downstream of antigen receptor engagement. A substantial number of studies have concentrated on the receptors themselves, and the enzymes stimulated in response to antigen binding (reviewed by Weiss & Littman, 1994; DeFranco, 1997).

Individual components of the T cell receptor (TCR) complex have been well characterised and in a number of cases the functionality of receptor sub-units or domains has been determined though the construction of chimeric receptor proteins (Kuwana *et al*., 1987; Romeo *et al*., 1992). Cytoplasmic signalling domains in particular, and their role in TCR activation, have been identified using this approach. However, more recently such chimeric receptors have been used as regulators of the cell activation process (see for example published International Patent Specifications WO 97/23613, and WO 95/02686). The ability to control the biological effects of cellular activation, for example, increased cellular proliferation, increased expression of cytokines, stimulation of cytolytic activity, differentiation of other effector functions, antibody secretion, phagocytosis, tumour infiltration and/or increased cellular adhesion, with chimeric receptors has considerable therapeutic potential.

Whilst currently available chimeric receptors are capable of effectively activating cells, there is room for improvement in the efficacy with which the cytoplasmic signalling domain of a chimeric receptor transduces the signal from the extracellular ligand-binding domain to downstream members of the secondary messenger pathway. such as members of the syk kinase and src-tyrosine kinase family members.

It would be an advantage if transduction through such a cytoplasmic signalling domain could be improved or manipulated in order to achieve a required level of effector cell activation. In addition it would also be extremely desirable to be able to activate a cell in a positive or negative way, thus inducing or inhibiting its biological functions with greater efficacy than is possible at present.

The current invention addresses these difficulties by providing nucleic acids encoding novel cytoplasmic signalling motifs and molecules, which, when expressed in effector cells, are capable of producing a range of desired cellular activation levels.

The term "cytoplasmic signalling sequence" as used herein, refers to cytoplasmic sequences of the TCR and co-receptors that act in concert to initiate signal transduction following antigen receptor engagement. The term also encompasses any derivative or variant of these sequences, and any synthetic sequence, that has the same functional capability. Signals generated through the TCR alone are insufficient for full activation of the T cell: a secondary or co-stimulatory signal is also required. Thus, cytoplasmic signalling components can be sub-divided into two classes: those that initiate antigen-dependent primary activation through the TCR (primary signalling sequences) and those that act in an antigen-independent manner to provide a secondary or co-stimulatory signal (secondary signalling sequences).

The current invention provides nucleic acids encoding novel cytoplasmic signalling sequences. which comprise non-natural stimulatory primary signalling motifs.

The term "primary signalling motif" is defined as a sequence that transduces either a stimulatory or an inhibitory signal, which regulates primary activation of the TCR complex. It will be appreciated that a primary signalling sequence may contain a single primary signalling motif or more than one primary signalling motif. inhibitory primary signalling motifs are exemplified by immunoreceptor tyrosine-based inhibitory motifs (ITIMs) and comprise the consensus amino acid sequence of I/V-X-Y-X₂-L (Burshtyn *et al*., 1999). Stimulatory primary signalling motifs conform to the consensus sequence Y-X₂-L/I-Xₙ-Y-X₂-L/I where the value of n lies between 6 and 8 and is most typically found to be 7. These motifs are also known as immunoreceptor tyrosine-based activation motifs or ITAMs, natural examples of which are described in a review by lsakov (1998).

In the aforementioned consensus sequences, and other similar formulae described herein, the standard single letter amino acid code is employed, X represents any amino acid, and a subscripted number indicates the number of residues present at that position within the consensus. It is intended that that the terms X₂ (or Xₙ) can represent 2 (or n) amino acids. which may either be the same or different.

This invention is based on novel non-natural sequences. which have been found unexpectedly to act as extremely efficient stimulatory primary signalling motifs. They are capable of modulating the level of primary signal transduction to different degrees. thus providing a range of cellular activation levels. It is known that primary activation involves the binding of src and the syk family of protein tyrosine kinase to ITAMs and the subsequent phosphorylation of tyrosine residues within the motifs. It is likely therefore, that these novel primary signalling motifs mediate primary signal transduction through having an altered specificity or affinity for these kinases. This can be analysed quite easily through assay by Western blot using an anti-phosphotyrosine antibody (Sanchez-Garcia, *et al*., 1997). Such an assay may be employed as the basis of a functional screen for the activity of novel sequences of the invention.

The non-natural stimulatory primary signalling motifs of this invention conform to the ITAM consensus sequence described above, and are based on. but not limited to the amino acid sequences: GQDGL**YQELNTRSRDEYSVL**EGRKAR designated SB14^{a} and GQDGL**YQELNTRSRDEAYSVL**EGRKAR designated SB15^{a}, (the position of the ITAM consensus sequence is emphasised in bold).

Thus according to the first aspect of the invention there is provided a nucleic acid encoding a non-natural stimulatory pnmary signalling motif comprising the amino acid sequence: GQDGLYQELNTRSRDEYSVLEGRKAR (designated SB14^{a}), or a variant thereof that differs in amino acid sequence in up to 11 positions and comprises the consensus sequence of Y-X₂-L/I-X₆₋₈-Y-X₂-L/I, wherein X represents any amino acid and a subscripted number indicates the number of residues present at that position within the consensus.

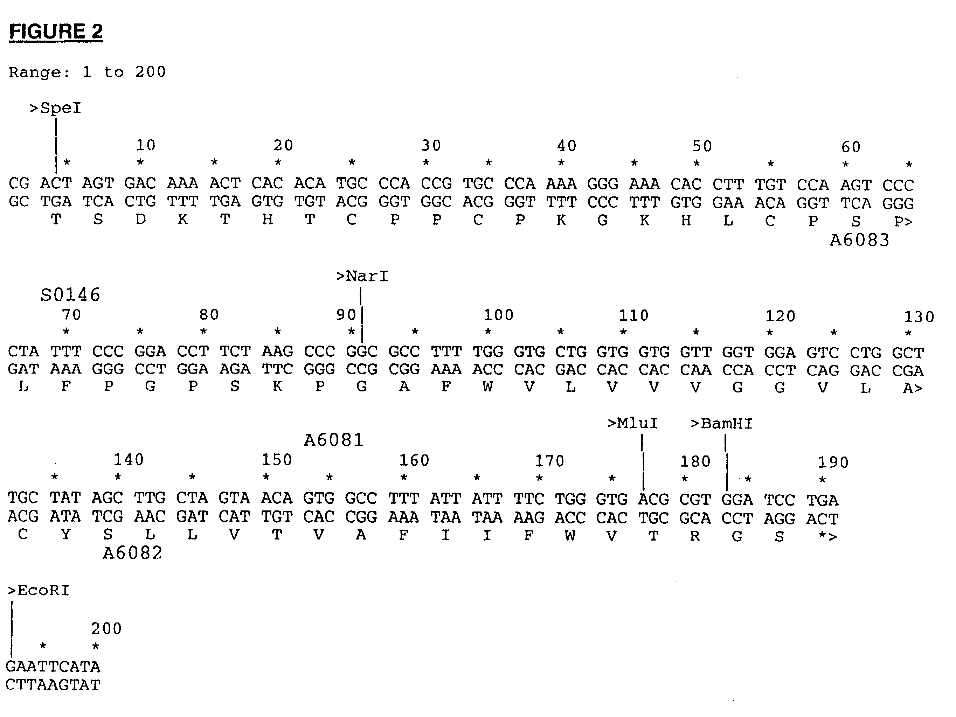

Other than in Figure 2 and in some instances in the examples (where the standard three-letter code is used to describe amino acid sequence), the standard single letter code is used throughout this application to describe both amino acid and nucleotide sequences.

Variant non-natural primary signalling motifs of the invention will contain at least one, and may contain between 1 and 11 vacations in sequence from SB14^{a} provided that the consensus sequence of Y-X₂-L/I-X₆₋₈-Y-X₂-L/I is maintained. A variation in sequence may correspond to the deletion. addition or substitution of any amino acid that is not vital for the maintenance of the consensus sequence. Preferably vanants will contain between 1 and 8 such variations and more preferably between 1 and 7 variations in sequence. One particularly preferred variant is GQDGLYQELNTRSRDEAYSVLEGRKAR (SB15^{a}). It is preferred that any variant of the invention is capable of signalling more efficiently than a known natural ITAM-containing sequence. The efficacy of such variants at mediating signalling may be measured in a number of ways. for example by assessing their interaction with downstream members of the secondary messenger pathway, such as members of the syk kinase and src-tyrosine kinase family members. This can be analysed quite easily through assay by Western blot using an anti-phosphotyrosine antibody (Sanchez-Garcia. *et al*., 1997). Alternatively, their efficacy at mediating signalling when incorporated in a chimeric receptor can be assessed easily as described herein in the Examples.

It has also been found that the magnitude of a signal transduced through an immune cell receptor may be tailored further, by employing additional cytoplasmic signalling motifs and/or sequences in conjunction with the novel stimulatory primary signalling motifs described above. Thus. in a second aspect of the invention, a nucleic acid is provided that encodes a cytoplasmic signalling molecule comprising a pnmary signalling motif according to the first aspect of the invention and at least one other primary signalling motif.

Stimulatory primary signalling motifs for use in this aspect of the invention will contain the consensus amino acid sequence Y-X₂-L/I-Xₙ-Y-X₂-L/I. Where the value of n lies between 6 and 8, it is preferred that at least one of the additional primary signalling motifs will be derived from all or part of a natural ITAM containing sequence, for example a primary signalling motif derived from the TCRζ chain. FcRγ, FcRβ, CD3γ, CD3δ, CD3ε, CD5, CD22, CD79a, CD79b or CD66d.

Examples of primary signalling motifs derived from these molecules are shown in Table 1 below. Preferably at the least one other primary signalling motif for use in the invention will be derived from all or part of TCRζ1, TCRζ2, TCRζ3, FcRγ, FcRβ, CD3γ, CD3δ, CD5, CD22, CD79a. CD79b or CD66d, or a variant thereof. However. it is equally preferred that the at least one other primary signalling motif will be a non-natural primary signalling motif according to the first aspect of the invention. Preferred examples of such non-natural primary signalling motifs are SB14^{a} or SB15^{a} as described herein.

Where it is intended that at least one further primary signalling motif acts as an inhibitory motif, it is preferred that the additional primary signalling motif comprises an ITIM containing sequence, i.e. it contains the consensus amino acid sequence of I/V-X-Y-X₂-L (Burshtyn *et al*., 1999).

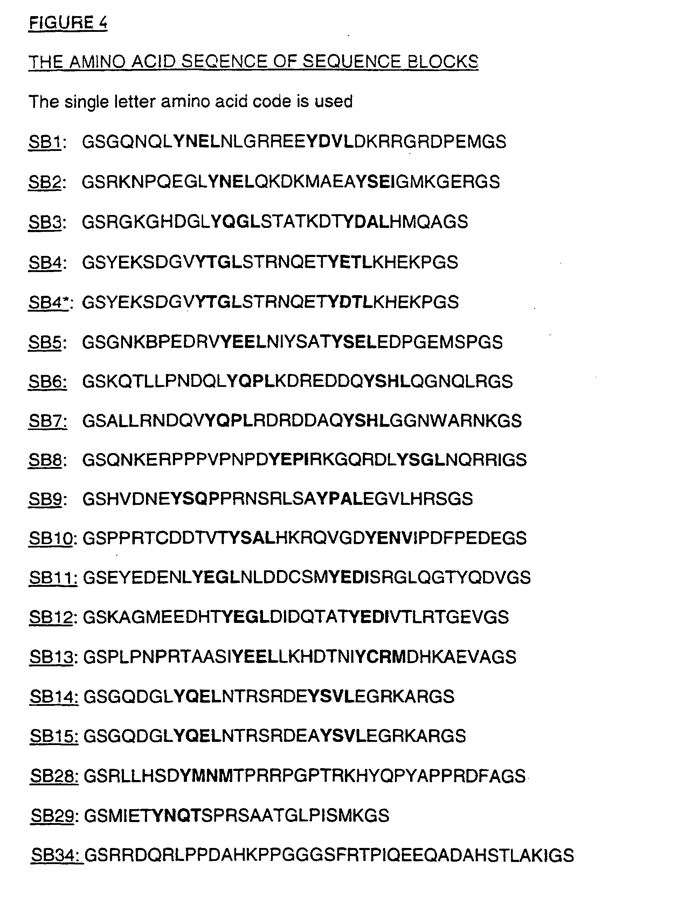

**Table 1. Source and amino acid sequences of primary signalling motifs of particular use in the invention.** The position of the consensus ITAM amino acid sequence is emphasised in bold. Figure 4 includes further examples of primary signalling motifs for use in the invention (SB1, SB2, SB3. SB4. SB4*, SB5, SB6, SB7, SB8, SB9, SB10, SB11, SB12, SB13. SB14. SB15). which correspond to the primary signalling motifs shown below with GS linkers incorporated at each end of the motif to facilitate cloning.

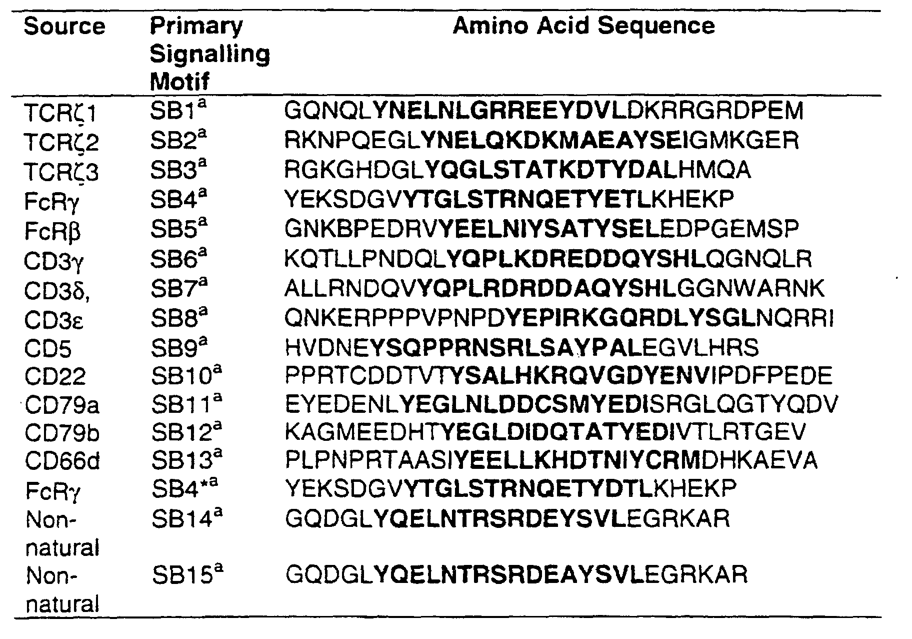

In a third aspect of the invention, a nucleic acid is provided that encodes a cytoplasmic signalling molecule comprising a primary signalling motif according to the first aspect of the invention and at least one secondary signalling sequence.

The term "secondary signalling sequences" means a sequence that imparts secondary or co-stimulatory signalling capacity to a molecule in T cells.

Molecules containing such sequences include CD2, CD4, CD8, CD28, CD134 and CD154 (see Finney *et al*., 1998). Preferred secondary signalling sequences for use in the invention are those derived from CD28. CD134 and CD154. for example. SB28^{a}. which has the amino acid sequence RLLHSDYMNMTPRRPGPTRKHYQPYAPPRDFA. SB29^{a}, which has the amino acid sequence MIETYNQTSPRSAATGLPISMK and SB34^{a}, which has the amino acid sequence RRDQRLPPDAHKPPGGGSFRTPIQEEQADAHS TLAKI. Further examples of secondary signalling sequences are shown in Figure 4 as SB28, SB29 and SB34, where GS linkers have been incorporated at each end of the sequence to facilitate cloning.

Novel cytoplasmic signalling sequences and molecules of the invention can be used either by themselves or as a component part of a larger protein, such as a chimeric receptor. As individual protein molecules, they can be introduced into, or expressed in, effector cells in order to act as substitute cytoplasmic signalling sequences for immune cell receptors already expressed within that cell. In this way they can increase the efficiency of signalling through the receptor. Alternatively, they may be introduced into a cell in order to compete with the existing src- and syk- protein kinase binding sites and thus, regulate the degree of cell activation by acting as protein kinase inhibitors. Any one of a number of *in vitro* assays may be used to estimate the efficacy of these novel cytoplasmic signalling molecules as protein tyrosine kinase inhibitors, see for example published International Patent application WO98/11095.

However. it is envisaged that the cytoplasmic signalling sequences and molecules of this invention are used preferentially to mediate signalling when employed as intracellular components of a chimeric receptor protein. Such chimeric receptors additionally compnse an extracellular ligand-binding domain and a transmembrane domain.

The incorporation of an extracellular ligand-binding domain confers on the chimenc receptor the ability to exhibit specificity for a specific ligand or class of ligands. This specificity can be used to define precise ligands or classes of ligands that are capable of activating the receptor. In this way the receptor may be designed to activate the cell in which it is expressed upon binding a chosen class of. or individual, ligand.

Contact between the ligand and its corresponding binding domain in a chimeric receptor. results in signal transduction through the cytoplasmic signalling domain. The choice of motif, or combination of motifs or motifs and sequences, within the cytoplasmic signalling domain, dictates the magnitude of the signal transduced, and consequently controls the level to which the cell is activated.

A further embodiment of the invention thus provides a nucleic acid encoding a chimeric receptor protein, which compnses an extracellular ligand-binding domain, a transmembrane domain, and a cytoplasmic signalling domain, wherein the cytoplasmic signalling domain is encoded by a nucleic acid according to any of the previously described aspects of the invention.

The term "extracellular ligand-binding domain" as used herein, is defined as any oligo- or polypeptide that is capable of binding a ligand. Accordingly antibody binding domains, antibody hypervariable loops or CDRs, receptor binding domains and other ligand binding domains. examples of which will be readily apparent to the skilled artisan, are described by this term. Preferably the domain will be capable of interacting with a cell surface molecule. Examples of proteins associated with binding to cell surface molecules that are of particular use in this invention include, antibody variable domains (V_{H} or V_{L}), T-cell receptor variable region domains (TCRα, TCRβ, TCRγ, TCRδ), or the chains of CD8α, CD8b, CD11A CD11B, CD11C, CD18. CD29, CD49A, CD49B, CD49D, CD49E, CD49F, CD61, CD41, or CD51. Whilst it may be of benefit to use the entire domain or chain in some instances, fragments may be used where appropriate.

Particularly useful binding components are derived from antibody binding domains and include Fab' fragments or. especially single chain Fv fragments.

The choice of domain will depend upon the type and number of ligands that define the surface of a target cell. For example. the extracellular ligand binding domain may be chosen to recognise a ligand that acts as a cell surface marker on target cells associated with a particular disease state. Thus examples of cell surface markers that may act as ligands include those associated with viral, bacterial and parasitic infections. autoimmune disease and cancer cells. In the latter case, specific examples of cell surface markers are the bombesin receptor expressed on lung tumour cells. carcinoembryonic antigen (CEA), polymorphic epithelial mucin (PEM), CD33. the folate receptor, epithelial cell adhesion molecule (EPCAM) and *erb*-B2. Other ligands of choice are cell surface adhesion molecules. inflammatory cells present in autoimmune disease. and T-cell receptors or antigens that give rise to autoimmunity. The potential ligands listed above are included by way of example; the list is not intended to be exclusive and further examples will be readily apparent to those of skill in the art.

Chimeric receptors may be designed to be bi- or multi-specific i.e. they may comprise more than one ligand binding domain and therefore, be capable of exhibiting specificity for more than one ligand. Such receptors may recruit cellular immune effector cells (e.g. T cells. B cells, natural killer (NK) cells, macrophages, neutrophils, eosinophils, basophils, or mast cells), or components of the complement cascade.

A further component of a chimeric receptor is the transmembrane domain. This may be derived either from a natural or from a synthetic source. Where the source is natural, the domain may be derived from any membrane-bound or transmembrane protein. Transmembrane regions of particular use in this invention may be derived from (i.e. comprise at least the transmembrane region(s) of) the α, β or ζ chain of the T-cell receptor, CD28, CD3ε, CD45, CD4, CD5, CD8, CD9, CD16, CD22, CD33, CD37, CD64, CD80, CD86, CD134, CD137 or CD154. It is preferable that the transmembrane domain is derived from all or part of the α, β or ζ chain of the T-cell receptor. CD28, CD3ε, CD45, CD4, CD5, CD8, CD9. CD16. CD22. CD33, CD37. CD64.

CD80, CD86, CD134, or CD154. The use of a transmembrane domain derived from CD28 is particularly preferred. Alternatively the transmembrane domain may be synthetic. in which case it will comprise predominantly hydrophobic residues such as leucine and valine (see for example Published International Patent Specification WO00/63374). Preferably a triplet of phenylalanine. tryptophan and valine will be found at each end of a synthetic transmembrane domain.

Between the extracellular ligand-binding domain and the transmembrane domain, or between the cytoplasmic signalling components and the transmembrane domain, there may be incorporated a spacer domain. As used herein, the term "spacer domain" generally means any oligo- or polypeptide that functions to link the transmembrane domain to, either the extracellular ligand-binding domain or, the cytoplasmic signalling components in the polypeptide chain. A spacer domain may comprise up to 300 amino acids, preferably 10 to 100 amino acids and most preferably 25 to 50 amino acids.

Spacer domains may be derived from all or part of naturally occurring molecules, such as from all or part of the extracellular region of CD8, CD4. or CD28; all or part of an antibody constant region: all or part of natural spacer components between functional parts of cytoplasmic signalling components, for example spacers between ITAMs may be used. Alternatively, the spacer may be an entirely synthetic spacer sequence.

Spacer domains may be designed in such a way that they, either minimise the constitutive association of chimeric receptors. thus reducing the incidence of constitutive activation in the cell or, promote such associations and enhance the level of constitutive activation in the cell. Either possibility may be achieved artificially by deleting, inserting, altering or otherwise modifying amino acids and naturally occurring sequences in the transmembrane and/or spacer domains, which have side chain residues that are capable of covalently or non-covalently interacting with the side chains of amino acids in other polypeptide cnains. Particular examples of amino acids that can normally be predicted to promote association include cysteine residues, charged amino acids or amino acids such as serine or threonine within potential glycosylation sites.

Cytoplasmic signalling motifs and sequences may be linked to each other, or to other intracellular components. or to the transmembrane domain. in a random or specified order. Optionally, a short oligo- or polypeptide linker, preferably between 2 and 10 amino acids in length may form the linkage. A glycine-serine doublet provides a particularly suitable linker.

Chimeric receptors may be designed in such a way that the spacer and transmembrane components have free thiol groups. thereby providing the receptor with multimerisation, and particularly dimerisation, capacity. Such multimeric receptors are preferred, especially dimers. Receptors with transmembrane and spacer domains derived from CD28 components, the zeta chain of the natural T cell receptor, and/or antibody hinge sequences are especially preferred.

It is intended that the component motifs and sequences of these novel cytoplasmic signalling molecules can be combined in any way so as to achieve the desired level of activation (or possible inhibition) of a number of secondary messenger cascades. When these cytoplasmic signalling sequences are employed as the intracellular domain of a chimeric receptor, this activation/inhibition may be initiated though a single binding event at the extracellular ligand-binding domain. It will be clear to the skilled technician that combinations of cytoplasmic signalling motifs and or sequences can be on separate polypeptide chains or may be in series on a single polypeptide chain. This concept of. either a single polypeptide or, multiple polypeptide chains providing the comprising elements. is equally applicable to the chimenc receptors of the present invention.

Thus the current invention also provides cytoplasmic signalling proteins and chimeric receptor proteins encoded by a nucleic acid as described in any of the previously described aspects of the invention.

Nucleic acid coding sequences for the novel primary signalling motifs, for use in this invention, are readily derived from the specified amino acid sequences. Other nucleic acid sequences are widely reported in the scientific literature and are also available in public databases. DNA may be commercially available. may be part of cDNA libraries. or may be generated using standard molecular biology and/or chemistry procedures as will be clear to those of skill in the art. Particularly suitable techniques include the polymerase chain reaction (PCR), oligonucleotide-directed mutagenesis, oligonucleotide-directed synthesis techniques. enzymatic cleavage or enzymatic filling-in of gapped oligonucleotide. Such techniques are described by Sambrook & Fritsch, 1989, and in the examples contained hereinafter.

The nucleic acids of the invention may be used with a carrier. The carrier may be a vector or other carrier suitable for the introduction of the nucleic acids *ex-vivo* or *in-vivo* into target cell and/or target host cells. Examples of suitable vector include viral vectors such as retroviruses, adenoviruses, adeno-associated viruses (AAVs), Epstein-Barr virus (EBV) and Herpes simplex virus (HSV). Non-viral vector may also be used, such as liposomal vectors and vectors based on condensing agents such as the cationic lipids described in International patent application numbers WO96/10038, WO97/18185, WO97/25329, WO97/30170 and WO97/31934. Where appropriate, the vector may additionally include promoter and regulatory sequences and/or replication functions from viruses, such as retrovirus long terminal repeats (LTRs), AAV repeats. SV40 and human cytomegalovirus (hCMV) promoters and/or enhancers. splicing and polyadenylation signals and EBV and BK virus replication functions. Tissue-specific regulatory sequences such as the TCR-α promoter. E-selectin promoter and the CD2 promoter and locus control region may also be used. The carrier may be an antibody.

The invention also includes cloning and expression vectors containing a nucleic acid according to any of the above-described aspects of the transcriptional and translation control sequences, for example. enhancer elements, promoter-operator regions, termination stop sequence, mRNA stability sequences. start and stop codons or ribosome binding sites, linked where appropriate in-frame with the nucleic acid molecules of the invention.

Additionally in the absence of a naturally effective signal peptide in the protein sequence, it may be convenient to cause recombinant cytoplasmic signalling proteins to be secreted from certain hosts. Accordingly, further components of such vectors may include nucleic acid sequences encoding secretion signalling and processing sequences.

Vectors according to the invention include plasmids and viruses (including both bacteriophage and eukaryotic viruses). Many expression systems suitable for the expression of heterologous proteins are well known and documented in the art. For example, the use of prokaryotic cells such as *Escherichia coli* to express heterologous polypeptides and polypeptide fragments is well established (see for example, Sambrook & Fritsch, 1989, Glover, 1995a). Similarly, eukaryotic expression systems have been well developed and are commonly used for heterologous protein expression (see for example, Glover, 1995b and O'Reilly *et al*., 1993). In eukaryotic cells. apart from yeasts, the vectors of choice are virus-based. Particularly suitable viral vectors include baculovirus-, adenovirus-, and vaccinia virus-based vectors.

Vectors containing the relevant regulatory sequences (including promoter. termination. polyadenylation, and enhancer sequences, marker genes) can either be chosen from those documented in the literature. or readily constructed for the expression of the proteins of this invention using standard molecular biology techniques. Such techniques, and protocols for the manipulation of nucleic acids. for example in the preparation of nucleic acid constructs, mutagenesis, sequencing, DNA transformation and gene expression, as well as the analysis of proteins, are described in detail in Ausubel *et al*., 1992 or Rees *et al*., 1993.

Suitable host cells for the *in vitro* expression of high levels of cytoplasmic signalling molecules or chimeric receptors include prokaryotic cells e.g. *E. coli*. eukaryotic yeasts e.g *Saccharomyces cerevisiae, Pichia* species, *Schizosaccharomyces pombe,* mammalian cell lines and insect cells. Alternatively recombinant proteins or chimeric receptors may be expressed *in vivo*, for example in insect larvae, or plant cells, or more preferably mammalian tissues.

Nucleic acid may be introduced into a host cell by any suitable technique. In eukaryotic cells these techniques may include calcium phosphate transfection, DEAE-Dextran, electroporation, particle bombardment. liposome-mediated transfection or transduction using retrovirus, adenovirus or other viruses, such as vaccinia or, for insect cells, baculovirus. In bacterial cells, suitable techniques may include calcium chloride transformation, electroporation or transfection using bacteriophage. The nucleic acid may remain in an episomal form within the cell, or it may integrate into the genome of the cell. If the latter is desired, sequences that promote recombination with the genome will be included in the nucleic acid. Following introduction of the nucleic acid into host cells, the cells may be cultured under conditions to enhance or induce expression of the recombinant cytoplasmic signalling molecule or chimeric receptor protein as appropriate.

Thus, further aspects of the invention provide host cells containing a nucleic acid encoding a novel cytoplasmic signalling protein and/or chimeric receptor protein of the invention. and host cells expressing such proteins.

According to still further aspects. the nucleic acids of the invention can be employed in either *ex-vivo* or the *in-vivo* therapies.

For *ex-vivo* use, the nucleic acid may be introduced into effector cells, removed from the target host, using methods well known in the art e.g. transfection, transduction (including viral transduction), biolistics, protoplast fusion, calcium phosphate mediated DNA transformation, electroporation, cationic lipofection, or targeted liposomes. The effector cells are then reintroduced into the host using standard techniques. Examples of suitable effector cells for the expression of the chimeric receptors of the present invention include cells associated with the immune system such as lymphocytes e.g. cytotoxic T-lymphocytes, tumour infiltrating lymphocytes. neutrophils, basophils, or T-helper cells, dendritic cells, B-cells, haematopoietic stem cells. macrophages. monocytes or NK cells. The use of cytotoxic T-lymphocytes is especially preferred.

The nucleic acid according to this aspect of the invention is particularly suitable for *in vivo* administration. In order to achieve this. the DNA may be in the form of a targeted carrier system in which a carrier as described above is capable of directing DNA to a desired effector cell. Examples of suitable targeted delivery systems include targeted naked DNA, targeted liposomes encapsulating and/ or complexed with the DNA, targeted retroviral systems and targeted condensed DNA such as protamine and polylysine-condensed DNA.

Targeting systems are well known in the art and include, for example, using antibodies or fragments thereof against cell surface antigens expressed on target cells in vivo such as CD8, CD16, CD4, CD3, selecting (e.g. E-selectin), CD5, CD7, CD24, and activation antigens (e.g. CD69 and IL-2R. Alternatively other receptor-ligand interactions can be used for targeting e.g. CD4 to target HIV_{gp}160-expressing target cells.

In general, the use of antibody-targeted DNA is preferred, particularly antibody-targeted naked DNA. antibody-targeted condensed DNA and especially antibody-targeted liposomes. Types of liposomes that may be used include for example pH-sensitive liposomes. where linkers that are cleaved at low pH may be used to link the antibody to the liposome. The nucleic acids of the present invention may also be targeted directly to the cytoplasm by using cationic liposomes, which fuse with the cell membrane. Liposomes for use in the invention may also have hydrophilic molecules, e.g. polyethylene glycol polymers, attached to their surface to increase their circulating half-life. There are many example in the art of suitable groups for attaching DNA to liposomes or other carriers; see for example International patent application numbers WO88/04924, WO90/09782. WO91/05545, WO91/05546. WO93/19738, WO94/20073 and WO94/22429. The antibody or other targetting molecule may be linked to the DNA. condensed DNA or liposome using conventional linking groups and reactive functional groups in the antibody. e.g. thiols or amines, and in the DNA or DNA-containing material.

Non-targeted carrier systems may also be usea. In these systems targeted expression of the protein is advantageous. This may be achieved, for example, by using T cell specific promoter systems such as the zeta promoter, CD2 promoter and locus control region. CD4, CD8 TCRα and TCRβ promoters, cytokine promoters, such as the IL2 promoter, and the perforin promoter.

It is intended that cytoplasmic signalling proteins and/or chimeric receptor proteins of the present invention, or the nucleic acids encoding them, be applied in methods of therapy of mammalian, particularly human, patients. Signalling molecules and chimeric receptor proteins generated by the present invention may be particularly useful in the treatment of a number of diseases or disorders. Such diseases or disorders may include those described under the general headings of infectious diseases. e.g. HIV infection: inflammatory disease/autoimmunity e.g. asthma, eczema; congenital e.g. cystic fibrosis, sickle cell anaemia: dermatologic, e.g. psoriasis; neurologic, e.g. multiple sclerosis: transplants e.g. organ transplant rejection. graft-versus-host disease: metabolic/idiopathic disease. e.g. diabetes: cancer.

For example. expression of a chimeric receptor on the surface of a T cell may initiate the activation of that cell upon binding of the ligand-binding domain to a ligand on a target cell. The ensuing release of inflammatory mediators stimulated by the activation of the signalling function of the receptor ensures destruction of the target cell.

When a chimeric receptor according to the present invention is expressed in an effector cell of the immune system. binding to target will activate the effector cell; downstream effects of this activation may also result in the destruction of the target cell. If the extracellular ligand-binding domain of the chimeric receptor exhibits specificity for a surface marker on an immune cell, effector cells may be recruited to the site of disease. Accordingly, expression of a chimeric receptor in a diseased cell will ensure its destruction.

The expression of multispecific chimeric receptor proteins, or more than one chimenc receptor (with different ligand specificities), within a single host cell, may confer dual functionality on the receptor. For example. binding of the chimeric receptor to its target may not only activate the effector cell itself, but may additionally attract other immune effectors to the site of disease. The target cell may thus be destroyed by the activation of the immune system.

A further aspect of the invention provides a composition comprising a cytoplasmic signalling protein and/or chimeric receptor protein of the invention, or nucleic acid(s) encoding such a protein, in conjunction with a pharmaceutically acceptable excipient.

Suitable excipients will be well known to those of skill in the an and may. for example. comprise a phosphate-buffered saline (e.g. 0.01 M phosphate salts, 0.138M NaCl. 0.0027M KCl, pH7.4), a liquid such as water, saline, glycerol or ethanol, optionally also containing mineral acid salts such as hydrochlorides, hydrobromides, phosphates, sulphates and the like: and the salts of organic acids such as acetates propionates, malonates, benzoates and the like. Auxiliary substances such as wetting or emulsifying agents, and pH buffering substances, may also be present. A thorough discussion of pharmaceutically acceptable excipients is available in Remington's Pharmaceutical Sciences (Mack Pub. Co.. N.J. 1991). Preferably, the compositions will be in a form suitable for parenteral administration e.g. by injection or infusion, for example by bolus injection or continuous infusion or particle-mediated injection. Where the composition is for injection or infusion, it may take the form of a suspension. solution or emulsion in an oily or aqueous vehicle and it may contain formulatory agents such as suspending, preservative, stabilising and/or dispersing agents. Alternatively, the composition may be in dry form. for reconstitution before use with an appropriate sterile liquid. For particle-mediated administration. DNA may be coated on particles such as microscopic gold particles.

A carrier may also be used that does not itself induce the production of antibodies harmful to the individual receiving the composition and which may be administered without undue toxicity. Suitable carriers are typically large, slowly metabolised macromolecules such as proteins. polysaccharides. polylactic acids, polyglycolic acids, polymeric amino acids. amino acid copolymers and inactive virus particles. Pharmaceutical compositions may also contain preservatives in order to prolong shelf life in storage.

If the composition is suitable for oral administration, the formulation may contain, in addition to the active ingredient additives such as starch (e.g. potato, maize or wheat starch, cellulose), starch derivatives such as microcrystalline cellulose, silica, various sugars such as lactose, magnesium carbonate and/or calcium phosphate. It is desirable that a formulation suitable for oral administration be well tolerated by the patient's digestive system. To this end. it may be desirable to include mucus formers and resins. It may also be desirable to improve tolerance by formulating the compositions in a capsule that is insoluble in the gastric juices. In addition, it may be preferable to include the composition in a controlled release formulation.

According to yet a further aspect of the invention, the use of novel cytoplasmic signalling proteins or chimeric receptor proteins of the invention, or of nucleic acids encoding such proteins. or of a pharmaceutical composition as described herein, in the manufacture of a medicament for the treatment or prevention of disease in humans or in animals is also provided.

The various aspects and embodiments of the present invention will now be illustrated in more detail by way of example. It will be appreciated that modification of detail may be made without departing from the scope of the invention.

### BRIEF DESCRIPTION OF THE FIGURES

- Figure 1: Schematic representation of the cloning cassette used in the construction of chimeric receptors with novel signalling components.
- Figure 2: Nucleotide and amino acid sequence of h.CD28 extracellular spacer and the human CD28 transmembrane region used in the construction of the cloning cassette describe in Figure 1.
- Figure 3: Oligonucleotide sequences used in the construction of chimeric receptors.
- Figure 4: Amino acid sequences of primary signalling motifs and secondary signalling sequences (SB's) employed in the construction of chimeric receptors.
- Figure 5: Antigen specific stimulation (by the addition of N.CD33 cells) of chimeric receptors having natural or non-natural primary signalling motifs.
- Figure 6: Antigen specific stimulation (by the addition of HL60 cells) of chimeric receptors having natural or non-natural primary signalling motifs.
- Figure 7: A comparison of the signalling ability of chimeric receptors comprising a non-natural primary signalling motif alone and a non-natural primary signalling motif in series with an additional, secondary signalling sequence. Antigen specific stimulation provided by N.CD33 cells.
- Figure 8: A comparison of the signalling ability of chimeric receptors comprising a non-natural primary signalling motif alone and a non-natural primary signalling motif in series with an additional. secondary signalling sequence. Antigen specific stimulation provided by HL60 cells.
- Figure 9: A companson of the signalling ability of natural and non-natural pnmary signalling motifs.
- Figure 10: Enhanced IL-2 production by Jurkat cells expressing chimeric receptors with multiple copies of a non-natural primary signalling motif, in response to stimulation by HL60 cells.

### EXAMPLES

### Example 1. Construction of the cloning vector, pHMF393

To facilitate construction of chimeric receptors with different binding, extracellular spacer, transmembrane and signaling components. a cloning cassette system was devised in pBluescript SK+ (Stratagene). This is a modification of our cassette system descnbed in published International Patent specification WO97/23613.

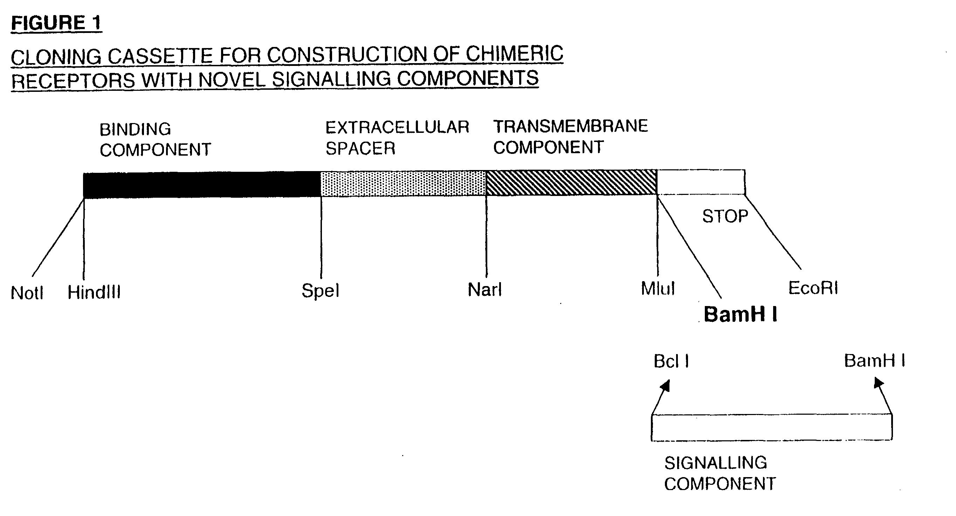

This new cassette system is shown in Figure 1. The binding component has 5' (relative to coding direction) Not I and Hind III restriction sites and a 3' (again relative to coding direction) Spe I restriction site. The extracellular spacer is flanked by a Spe I site (therefore encoding Thr, Ser at the 5' end) and a Nar I site (therefore encoding Gly, Ala at the 3' end). The transmembrane component is flanked by a Nar I site at its 5' end (therefore encoding Gly, Ala) and by Mlu I (therefore encoding Thr. Arg) and BamH I sites (therefore encoding Gly, Ser) at the 3' end. The signalling component may be cloned in-frame into the BamH I site. Following this BamH I site there is a stop codon for transcription termination and there is also an EcoR I site situated downstream of this to facilitate the subsequent rescue of whole constructs.

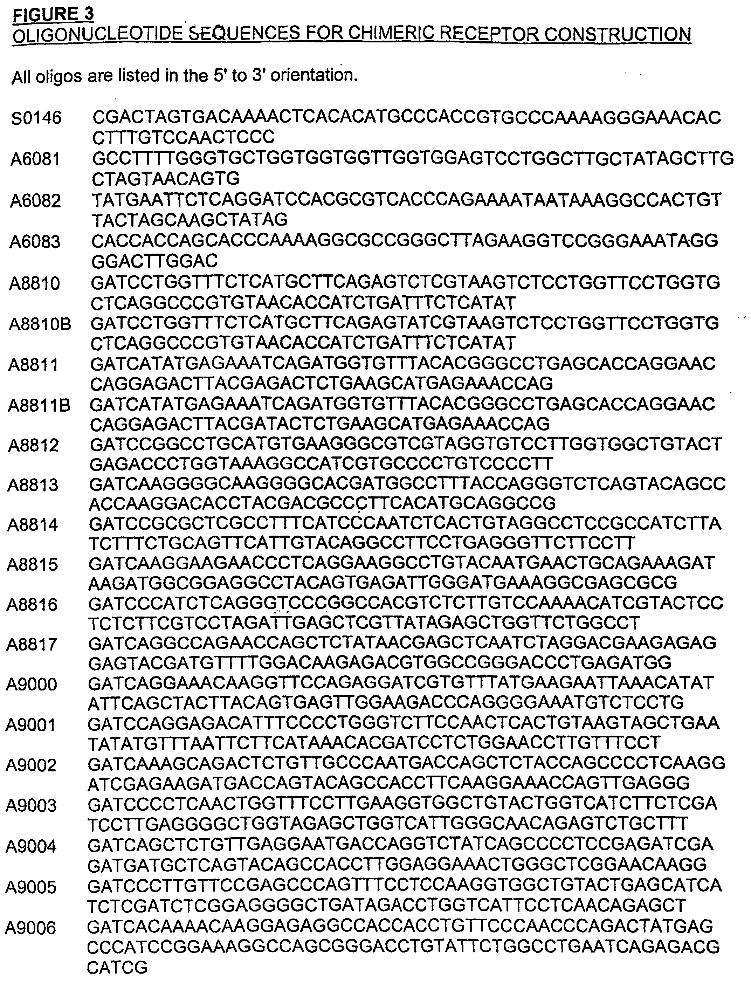

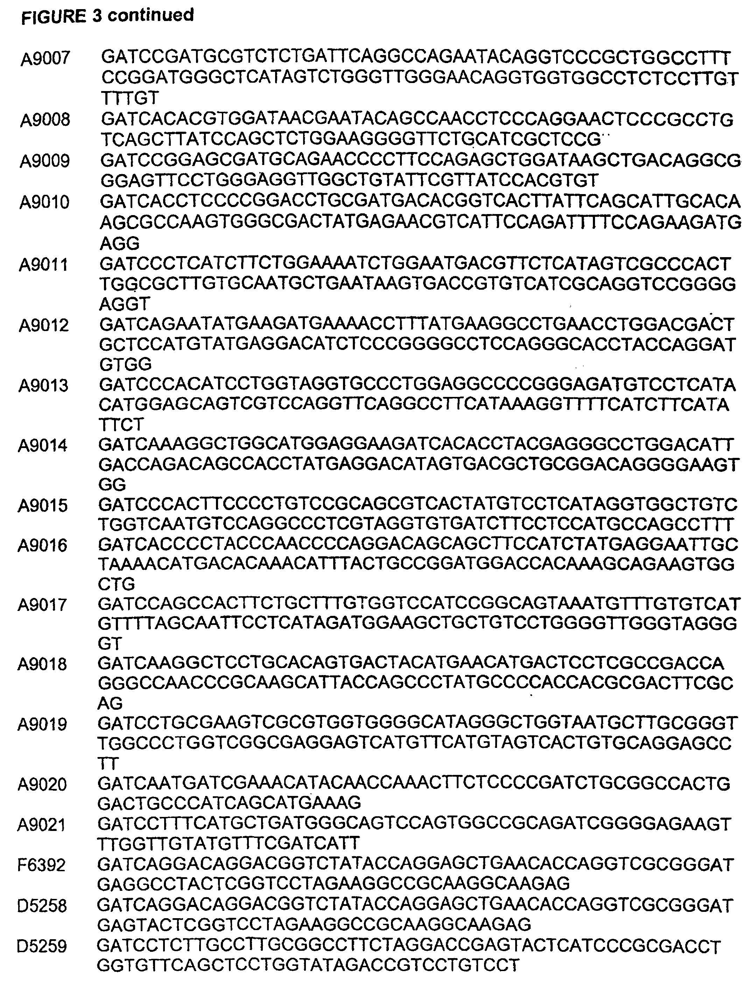

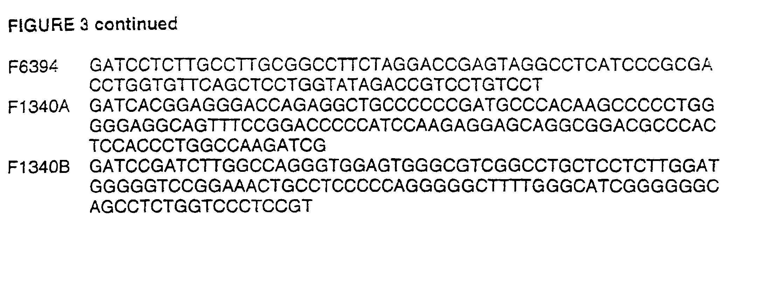

To generate the cassette. a 200bp fragment was assembled by PCR. using the following oligos: S0146, A6081. A6082 and A6083 (Figure 3). The nucleotide and amino acid sequences of this fragment are shown in Figure 2. It starts with a Spel site and consists of the extracellular spacer h.CD28. the human CD28 transmembrane region. a stop codon and finishes with an EcoR I restriction site. This PCR fragment was then digested with Spe I and EcoR I and substituted for the same fragment in our previously described cloning cassette system (Figure 2 of published International Patent application WO 97/23613) in order to clone it in-frame with the binding component.

### Example 2. The construction of sequence blocks of primary and secondary signalling motifs

Each sequence block (SB) was generated by annealing two oligos such that they had single-stranded overhangs forming half a Bcl I site at the 5' end and half a BamH I site at the 3' end. Oligos were annealed at a concentration of 1 pmole/µl in buffer (25mM NaCl. 12.5 mM Tris-HCl, 2.5mM MgCl₂, 0.25mM DTE, pH7.5) by heating in a boiling water bath for 5 minutes and then allowing them to cool slowly to room temperature.

The predicted amino acid sequences of these examples of SBs are shown in Figure 4 and sequence of the oligonucleotides employed in their construction are given in Figure 3. All oligonuiceotides were phosphorylated at their 5' end.
a) SB1: This sequence is based on the first ITAM of human TCRζ and was constructed by annealing oligos A8816 and A8817.
b) SB2: This sequence is based on the second ITAM of human TCRζ and was constructed by annealing oligos A8814 and A8815.
c) SB3: This sequence is based on the third ITAM of human TCRζ and was constructed by annealing oligos A8812 and A8813.
d) SB4: This sequence is based on the ITAM of the γ chain of human FcεR1 and was constructed by annealing oligos A8810 and A8811.
e) SB4*: This sequence was onginally generated in error by mis-annealment of the above oligos but was subsequently made by annealing oligos A8810B and A8811B.
f) SB5: This sequence is based on the ITAM of the β chain of human FcεR1 and was constructed by annealing oligos A9000 and A9001.
g) SB6: This sequence is based on the ITAM of the γ chain of human CD3 and was constructed by annealing oligos A9002 and A9003.
h) SB7: This sequence is based on the ITAM of the δ chain of human CD3 and was constructed by annealing oligos A9004 and A9005.
i) SB8: This sequence is based on the ITAM of the ε chain of human CD3 and was constructed by annealing oligos A9006 and A9007.
j) SB9: This sequence is based on the ITAM of human CD5 and was constructed by annealing oligos A9008 and A9009.
k) SB10: This sequence is based on the ITAM of human CD22 and was constructed by annealing oligos A9010 and A9011.
l) SB11: This sequence is based on the ITAM of human CD79a and was constructed by annealing oligos A9012 and A9013.
m) SB12: This sequence is based on the ITAM of human CD79b and was constructed by annealing oligos A9014 and A9015.
n) SB13: This sequence is based on the ITAM of human CD66d and was constructed by annealing oligos A9016 and A9017.
o) SB14: This sequence is synthetic and was constructed by annealing oligos D5258 and D5259.
p) SB15: This seauence is synthetic and was constructed by annealing oligos F6392 and F6394.
q) SB28: This sequence is based on the secondary (costimulation) sequence of human CD28 and was constructed by annealing oligos A9018 and A9019.
r) SB29: This sequence is based on the secondary (costimulation) sequence of human CD154 and was constructed by annealing oligos A9020 and A9021.
s) SB34: This sequence is based on the secondary (costimulation) signalling sequence of human CD134 and was constructed by annealing oligos F1340A and F1340B

### Example 3. The construction of receptors with different signalling components

Primary signalling motifs and secondary signalling sequences (SBs, see Figure 4) on a Bcl I to BamH I fragment may be cloned into the BamH I site of the cassette described in Example 1, downstream of the transmembrane region. Cloning in the correct orientation allows the subsequent downstream insertion of other pnmary signalling motifs and/or secondary signalling sequences.

This cassette also facilitates exchange of binding components on a Not I or Hind III to Spe I fragment as well as the exchange of extracellular spacers on a Spe I to Nar I fragment and the exchange of the transmembrane region on a Nar I to Mlu I fragment. Thus, chimeric receptors with different binding, extracellular spacer. transmembrane and signalling components can be assembled readily.

### Example 4. Analysis of chimeric receptors

a) Construction of expression plasmids. The chimeric receptor constructs were subcloned from pBluescript KS+ into the expression vector pEE6hCMV.ne (Cockett. *et al*., 1991) on a Hind III to EcoR I restriction fragment. The empty expression vector (i.e the base vector lacking in chimeric receptor genes) is used as a negative control.
b) Transfection into Jurkat E6.1 cells. To generate stable cell lines, the expression plasmids were linearised and transfected into Jurkat E6.1 cells (ECACC) by electroporation using a BioRad Gene Pulser. 10µg of DNA per 2.5 X10⁶ cells was given two pulses of 1000V. 3µF in 1ml PBS. Cells were left to recover overnight in non-selective media before being selected and cultured in media supplemented with the antibiotic G418 (Sigma) at 1.5mg/ml. After approximately four weeks cells were ready for analysis.
   For transient expression in Jurkat cells, the expression plasmids were transfected using DuoFect (Quantum Biotechnologies Inc.) according to the supplier's instructions.
c) Analysis of surface expression : FACS. Approximately 5X10⁵ Jurkat cells were stained with 1µg/ml FITC labelled antigen or antibody specific for the binding component of the chimeric receptor. For analysis of receptors with a P67scFV binding component, FITC-labelled CD33 antigen was employed. Fluorescence was analysed by a FACScan cytometer (Becton Dickinson).
d) Analysis of function : IL-2 production. 2X10⁵ cells were incubated at 37°C in 8% CO₂ for 20 hours in 96 well plates with target cells at an effector:target ratio of 1:1 . Cell supernatants were then harvested and assayed for human IL-2 (R & D Systems Quantikine kit).

Where P67scFv was employed as the example of an extracellular binding domain, the target cells used were:
HL60 cells - a human cell line naturally expressing the antigen. CD33
N.EE6 - a mouse myeloma (NS0) transfected with a control expression vector. These cells are used as a negative control target cell line.
N.CD33 - a mouse myeloma (NS0) transfected with an expression vector facilitating the expression of antigen CD33 on the ceil surface.

### Example 5. Results

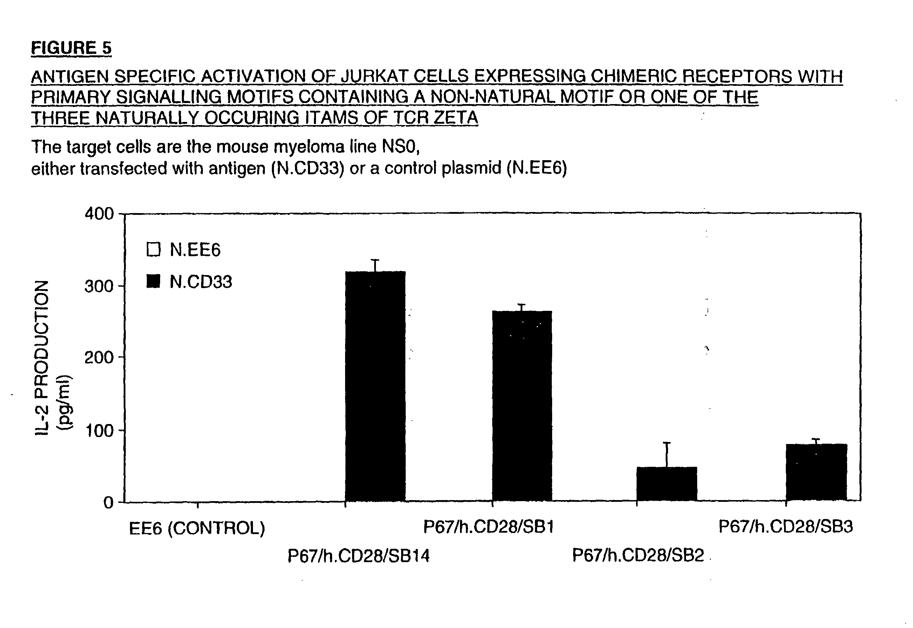

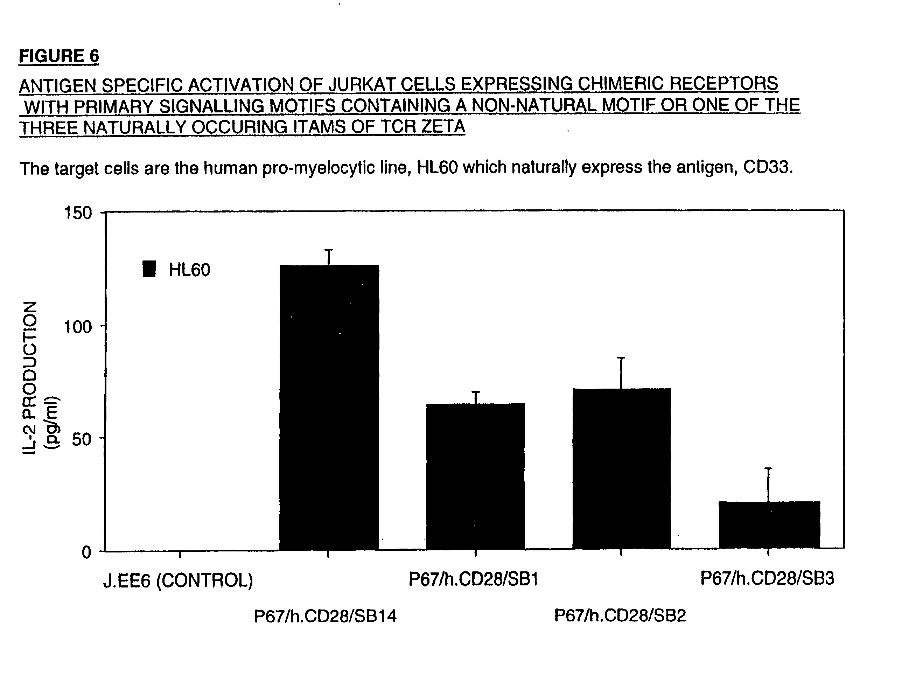

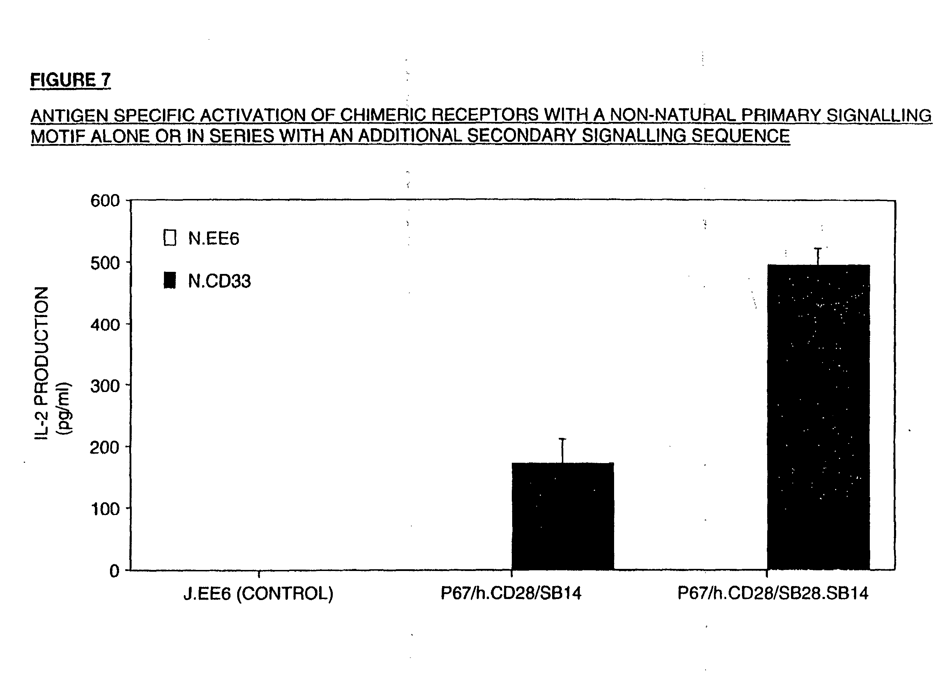

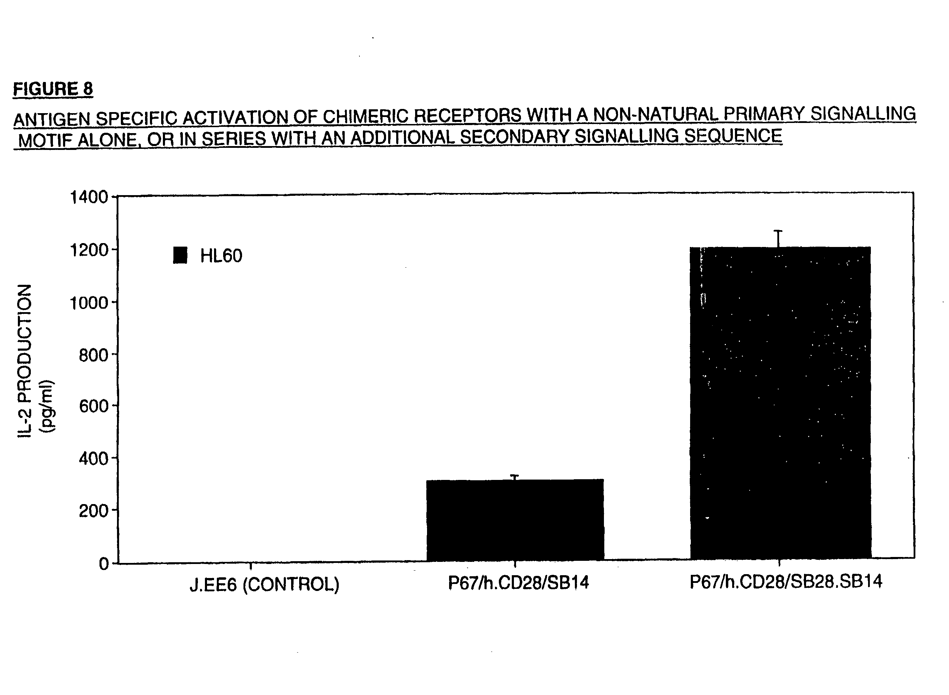

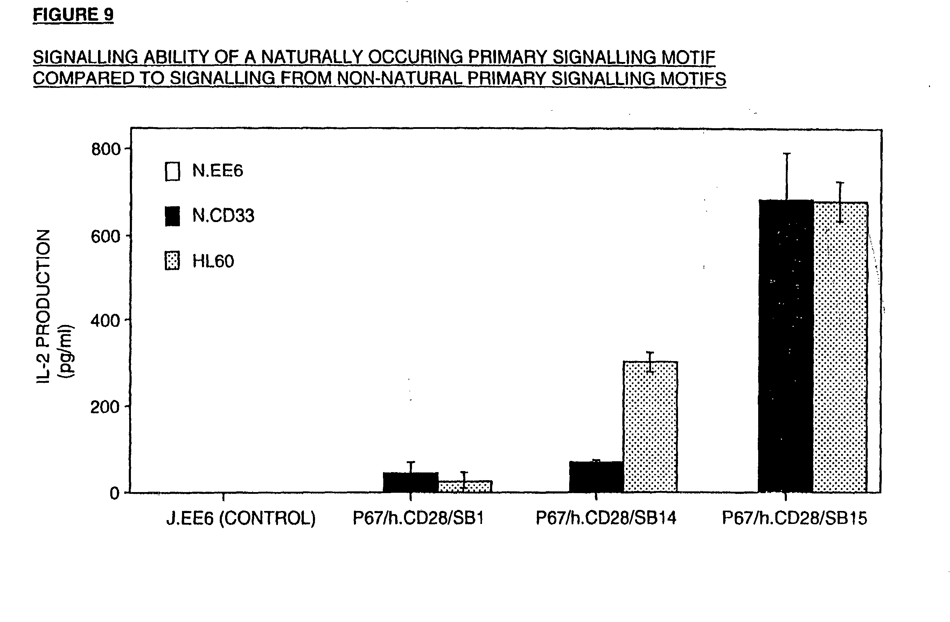

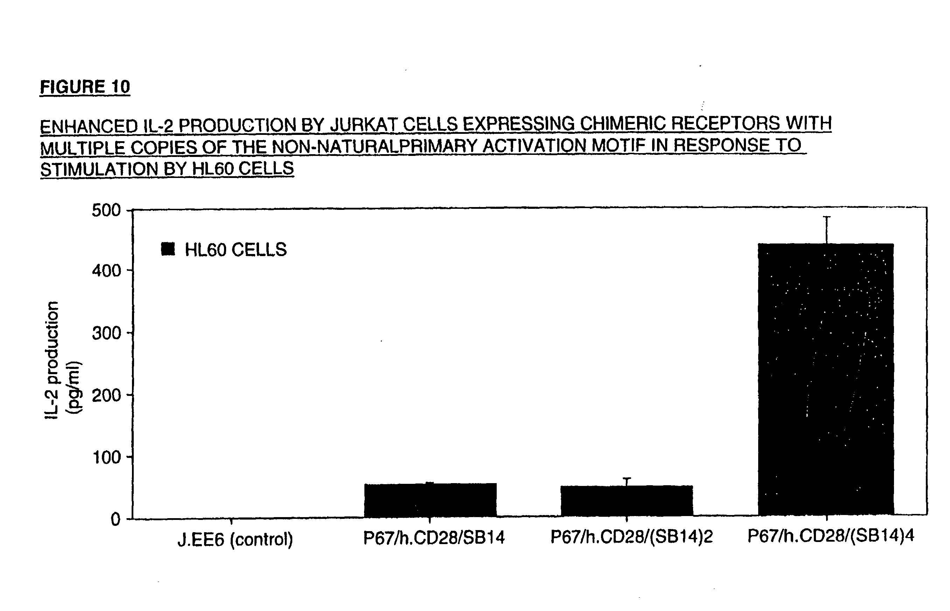

The specific production of IL-2 by Jurkat cells expressing chimeric receptors. in resoonse to antigen cnallenge (either by HL-60 or N.CD33 cells as indicated), is used as a measure of signalling capability and the degree of cellular activation in all of the experiments described below.
a) Comparison of the signalling capability of natural or non-natural primary signalling motifs (Figure 5). Jurkat cells expressing chimeric receptors with natural primary signalling motifs (P67scFV/h.CD28/SB1. P67scFV/h.CD28/SB2. P67scFV/h.CD28/SB3 derived from the TCR zeta chain) and non-natural primary signalling motifs (P67scFV/h.CD28/SB14) were challenged with N.CD33 cells. The resulting levels of IL-2 production are compared in Figure 5. All chimeric receptors are capable of producing IL-2 in response to a specific stimulus, i.e. the CD33 antigen. however the non-natural primary signalling motif quite clearly out-performs the individual natural ITAMs of TCRζ.
b) Further exemplification that SB14^{a}, a non-natural primary signalling motif, is more efficacious than the natural primary signalling motifs of the TCR zeta chain (Figure 6). Figure 6 illustrates the result of a similar experiment to that illustrated in Figure 5. except that in this particular instance the antigen challenge is provided by HL60 cells. The results clearly show that the non-natural primary signalling motif is far more efficacious than the individual natural ITAMs of TCRζ.
c) Signalling by the chimeric receptor P67scFV/h.CD28/SB14 can be enhanced by the inclusion of an additional, secondary signalling sequence (Figure 7). This demonstrates the specific IL-2 production by Jurkat cells expressing chimeric receptors containing a non-natural primary signalling motif in response to antigen provided by N.CD33 cells. The result shows the advantage of including a secondary activation motif in series with the non-natural primary activation motif.
d) Further exemplification that the incorporation of an additional, secondary signalling sequence is advantageous (Figure 8). Figure 8 illustrates the result of a similar experiment to that illustrated in Figure 7. except that in this particular instance the antigen challenge is provided by HL60 cells. It is again clear that the inclusion of a secondary signalling sequence in series with the non-natural primary signalling motif leads to more efficient signalling and an enhanced level of IL-2 production.
e) Signalling by chimeric receptors with non-natural primary signalling motifs is more efficient than by those with a natural primary signalling motif (Figure 9). The ability of Jurkat cells. expressing chimeric receptors comprising natural (TCRζ1 ITAM) or non-natural (SB14^{a} or SB15^{a}) primary signalling motifs, to produce IL-2 in response to antigen challenge by either N.CD33 or HL60 cells. is compared in Figure 9. These results show that both non-natural primary signalling motifs are more efficient the most potent individual natural primary signalling motif of the TCRζ chain, the TCRζ1 ITAM.
f) Signalling by chimeric receptors with a non-natural primary signalling motif can be enhanced by the inclusion of additional, primary, signalling motifs (Figure 10). Jurkat cells expressing chimeric receptors containing one or more non-natural primary signalling motifs were challenged with HL60 cells. The results in Figure 10 show the advantage of including additional primary signalling motifs, in this case multiple copies of a non-natural primary signalling motif, within the cytoplasmic signalling domain of a chimeric receptor.

### REFERENCES CITED

Ausubel, F.W. & Kingston R.E. (1992) *Short protocols in Molecular Biology.* 2nd edition. John Wiley & Sons Ltd.

Burshtyn. D.N., Lam. A.S.. Weston. M.. Gupta. N., Warmerdam. P.A. & Long, E.O. (1999) *Journal of Immunology* 162:897-902.

Cockett, M.I., Bebbington, C.R. & Yarranton, G.T. (1991) *Nucleic Acids Research* 19:319-325.

DeFranco A.L. (1997) *Current Opinion in Immunology* 9:296-308. Finney, H.M.. Lawson. A.D.G.. Bebbington, C.R. & Weir, A.C.N. (1998) *Journal of Immunology* 161:2791-2797.

Glover. D.M. 1995a. *DNA cloning: a practical approach. Volume II:*

*Expression systems*. IRL press.

Glover, D.M. 1995b. *DNA cloning: a practical approach. Volume IV:*

*Mammalian systems.* IRL press.

Kuwana, Y.. Asakura. Y.. Utsunomiya, N., Nakanishi, M.. Arat, Y.. Itoh. S..

Nagese, F. & Kurosawa. Y. (1987) *Biochemical and Biophysical Research Communications* 149:960-968.

O'Reilly, D.R.. & Miller, L.K. (1993). *Baculovirus expression vectors- A laboratory manual.* Oxford University Press.

Rees. A.R (1993) *Protein Engineering: a practical approach.* IRL press

Romeo. C.. Kolanus. W.. Amiot. M. & Seed. B. (1992) *Cold Spring Harbour*

*Symposia on Quantitative Biology*. Volume LVII pp 117-125.

Sambrook, J., & Fritsch, E. (1989) *Molecular cloning: a laboratory manual*. 2nd edition. Cold Spring Harbour Press. N.Y.

Sancez-Garcia, F.J.. Aller, W.W. & McCormack, W.T. (1997) *Immunology* 91:81-87.

Weiss. A. & Littman. D.R. (1994) *Cell* 76:26-274.

## Claims

1. A nucleic acid encoding a non-natural stimulatory primary signalling motif comprising the amino acid sequence: or a variant thereof that differs in amino acid sequence in up to 11 positions and comprises the consensus sequence of X₂-L/I-X₆₋₈-Y-X₂-L/I. wherein X represents any amino acid and a subscripted number indicates the number of residues present at that position within the consensus.

2. A nucleic acid encoding a variant of SB14^{a} according to claim 1. wherein the variant comprises at least one variation in amino acid sequence from SB14^{a}.

3. A nucleic acid encoding a variant of SB14^{a} according to claim 2, wherein the variant comprises between 1 and 8 variations in amino acid sequence from SB14^{a}.

4. A nucleic acid encoding a variant of SB14^{a} according to claim 3, wherein the variant comprises between 1 and 7 variations in amino acid sequence from SB14^{a}.

5. A nucleic acid according to any one of the preceding claims. wherein the variant is SB15^{a}, which has the amino acid sequence

6. A nucleic acid according to claim 1. wherein the non-natural stimulatory primary signalling motif is SB14^{a} which has the amino acid sequence:

7. A nucleic acid encoding a cytoplasmic signalling molecule, which comprises a non-natural stimulatory primary signalling motif according to any one of the preceding claims and at least one other primary signalling motif.

8. A nucleic acid according to claim 7, wherein the at least one other primary signalling motif is an immunoreceptor tyrosine-based activation motif (ITAM).

9. A nucleic acid according to claim 8. wherein the at least one other primary signalling motif is derived from the ζ chain of the TCR, FcRγ, FcRβ, CD3γ, CD3δ, CD3ε, CD5, CD22, CD79a, CD79b or CD66d.

10. A nucleic acid according to claim 7, wherein the at least one other primary signalling motif is a non-natural primary signalling motif as defined in any one of claims 1 to 6.

11. A nucleic acid according to claim 10, wherein the non-natural primary signalling motif is SB14^{a} or SB15^{a} as defined in claims 6 and 5 respectively.

12. A nucleic acid according to claim 7, wherein the at least one other primary signalling motif is an immunoreceptor tyrosine based inhibition motif (ITIM).

13. A nucleic acid encoding a cytoplasmic signalling molecule, which comprises a non-natural stimulatory primary signalling motif according to any one of claims 1 to 6 and at least one secondary signalling sequence.

14. A nucleic acid according to claim 13, wherein the at least one secondary signalling sequence is derived from CD28. CD134 or CD154.

15. A nucleic acid according to claim 14. wherein the at least one secondary signalling sequence is RLLHSDYMNMTPRRPGPTRKHYQPYAPPRDFA, MIETYNQTSPRSAATGLPISMK, or RRDQRLPPDAHKPPGGGSFRTPIQEEQADAHSTLAKI.

16. A nucleic acid encoding a cytoplasmic signalling molecule, which comprises a non-natural stimulatory motif according to any one of claims 1 to 6 and at least one other primary signalling motif and at least one secondary signalling sequence.

17. A nucleic acid encoding a chimeric receptor protein, which comprises an extracellular ligand-binding domain, a transmembrane domain, and a cytoplasmic signalling domain, wherein the cytoplasmic signalling domain is encoded by a nucleic acid according to any one of the preceding claims.

18. A nucleic acid according to claim 17, wherein the extracellular ligand-binding domain is an antibody binding domain, or a fragment thereof.

19. A nucleic acid according to claim 18, wherein the extracellular ligand-binding domain is a Fab' fragment or a scFv.

20. A nucleic acid according to claim 19, wherein the extracellular-ligand binding domain is a scFv.

21. A nucleic acid according to any one of claims 17 to 20. wherein the transmembrane domain is derived from the α, β or ζ chain of the T-cell receptor, CD28, CD3ε, CD45, CD4, CD5, CD8, CD9, CD16, CD22, CD33, CD37, CD64, CD80, CD86, CD134, CD137 or CD154.

22. A nucleic acid according to claim 21. wherein the transmembrane domain is derived from CD28.

23. A nucleic acid according to any one of claims 17 to 20. wherein the transmembrane domain is synthetic.

24. A vector comprising a nucleic acid according to any one of the preceding claims.

25. A cytoplasmic signalling protein encoded by a nucleic acid according to any one of claims 1 to 16.

26. A chimeric receptor protein encoded by a nucleic acid according to any one of claims 17 to 23.

27. A nucleic acid, a cytoplasmic signalling protein, or a chimeric receptor protein according to any one of the preceding claims, for use in therapy.

28. A composition comprising a nucleic acid, a cytoplasmic signalling protein. or a chimeric receptor protein according to any one of the preceding claim. in conjunction with a pharmaceutically acceptable excipient.

29. The use of nucleic acid, or of a cytoplasmic signalling protein, or of a chimeric receptor protein, or of a composition, according to any one of the preceding claims, in the manufacture of a medicament for the treatment or prevention of disease in humans or in animals.

30. A host cell containing a nucleic acid according to any one of claims 1 to 23. or a vector according to claim 24.

31. A host cell expressing a cytoplasmic signalling protein according to claim 25, or a chimenc receptor protein according to claim 26.

## Patentansprüche

1. Nukleinsäure, welche ein nicht natürliches stimulatorisches primäres Signalmotiv kodiert, umfassend die Aminosäuresequenz oder eine Variante davon, welche sich in der Aminosäuresequenz in bis zu 11 Positionen unterscheidet und die Konsensus-Sequenz von X₂-L/I-X₆₋₈-Y-X₂-L/I umfaßt, wobei X eine Aminosäure darstellt und eine tiefgestellte Zahl die Anzahl der Reste, welche an dieser Position innerhalb des Konsensus vorhanden sind, bezeichnet.

2. Nukleinsäure, welche eine Variante von SB14^{a} nach Anspruch 1 kodiert, wobei die Variante mindestens eine Veränderung in der Aminosäuresequenz von SB14^{a} umfaßt.

3. Nukleinsäure, welche eine Variante von SB14^{a} nach Anspruch 2 kodiert, wobei die Variante zwischen 1 und 8 Veränderungen in der Aminosäuresequenz von SB14^{a} umfaßt.

4. Nukleinsäure, welche eine Variante von SB14^{a} nach Anspruch 3 kodiert, wobei die Variante zwischen 1 und 7 Veränderungen in der Aminosäuresequenz von SB14^{a} umfaßt.

5. Nukleinsäure nach einem der vorhergehenden Ansprüche, wobei die Variante SB15^{a} ist, welche die Aminosäuresequenz GQDGLYQELNTRSRDEAYSVLEGRKAR aufweist.

6. Nukleinsäure nach Anspruch 1, wobei das nicht natürliche stimulatorische primäre Signalmotiv SB14^{a} ist, welches die Aminosäuresequenz GQDGLYQELNTRSRDEYSVLEGRKAR aufweist.

7. Nukleinsäure, welche ein cytoplasmatisches Signalmolekül kodiert, welches ein nicht natürliches stimulatorisches primäres Signalmotiv nach einem der vorhergehenden Ansprüche und mindestens ein anderes primäres Signalmotiv umfaßt.

8. Nukleinsäure nach Anspruch 7, wobei das mindestens eine andere primäre Signalmotiv ein immunrezeptor Tyrosin-basierendes Startmotiv (ITAM) ist.

9. Nukleinsäure nach Anspruch 8, wobei das mindestens eine andere primäre Signalmotiv von der ζ-Kette des TCR, FcRγ, FcRβ, CD3γ, CD3δ, CD3ε, CD5, CD22, CD79a, CD79b oder CD66d abgeleitet ist.

10. Nukleinsäure nach Anspruch 7, wobei das mindestens eine andere primäre Signalmotiv ein nicht natürliches primäres Signalmotiv, wie in einem der Ansprüche 1 bis 6 definiert, ist.

11. Nukleinsäure nach Anspruch 10, wobei das nicht natürliche primäre Signalmotiv SB14^{a} oder SB15^{a}, wie in einem der Ansprüche 6 bzw. 5 definiert, ist.

12. Nukleinsäure nach Anspruch 7, wobei das mindestens eine andere primäre Signalmotiv ein Immunrezeptor Tyrosin-basierendes Hemmmotiv (ITIM) ist.

13. Nukleinsäure, welche ein cytoplasmatisches Signalmolekül kodiert, welches ein nicht natürliches stimulatorisches primäres Signalmotiv nach einem der Ansprüche 1 bis 6 und mindestens eine zweite Signalsequenz umfaßt.

14. Nukleinsäure nach Anspruch 13, wobei die mindestens eine zweite Signalsequenz von CD28, CD134 oder CD154 abgeleitet ist.

15. Nukleinsäure nach Anspruch 14, wobei die mindestens eine zweite Signalsequenz RLLHSDYMNMTPRRPGPTRKHYQPYAPPRDFA, MIETYNQTSPRS-AATGLPlSMK oder RRDQRLPPDAHKPPGGGSFR-TPIQEEQADAHSTLAKI ist.

16. Nukleinsäure, welche ein cytoplasmatisches Signalmolekül kodiert, welches ein nicht natürliches stimulatorisches Motiv nach einem der Ansprüche 1 bis 6 und mindestens ein anderes primäres Signalmotiv und mindestens eine zweite Signalsequenz umfaßt.

17. Nukleinsäure, welche ein chimäres Rezeptorprotein kodiert, welches eine extrazelluläre Ligandenbindungsdomäne, eine Transmembrandomäne und eine cytoplasmatische Signaldomäne umfaßt, wobei die cytoplasmatische Signaldomäne durch eine Nukleinsäure nach einem der vorhergehenden Ansprüche kodiert ist.

18. Nukleinsäure nach Anspruch 17, wobei die extrazelluläre Ligandenbindungsdomäne eine Antikörperbindungsdomäne oder ein Fragment davon ist.

19. Nukleinsäure nach Anspruch 18, wobei die extrazelluläre Ligandenbindungsdomäne ein Fab'Fragment oder ein scFv ist.

20. Nukleinsäure nach Anspruch 19, wobei die extrazelluläre Ligandenbindungsdomäne ein scFv ist.

21. Nukleinsäure nach einem der Ansprüche 17 bis 20, wobei die Transmembrandomäne von der α-, β- oder ζ-Kette des T-Zell-Rezeptors, CD28, CD3ε, CD45, CD4, CD5, CD8, CD9, CD16, CD22, CD33, CD37, CD64, CD80, CD86, CD134, CD137 oder CD154 abgeleitet ist.

22. Nukleinsäure nach Anspruch 21, wobei die Transmembrandomäne von CD28 abgeleitet ist.

23. Nukleinsäure nach einem der Ansprüche 17 bis 20, wobei die Transmembrandomäne synthetisch ist.

24. Vektor, welcher eine Nukleinsäure nach einem der vorhergehenden Ansprüche umfaßt.

25. Cytoplasmatisches Signalprotein, welches durch eine Nukleinsäure nach einem der Ansprüche 1 bis 16 kodiert ist.

26. Chimäres Rezeptorprotein, welches durch eine Nukleinsäure nach einem der Ansprüche 17 bis 23 kodiert ist.

27. Nukleinsäure, ein cytoplasmatisches Signalprotein oder ein chimäres Rezeptorprotein nach einem der vorhergehenden Ansprüche zur Verwendung in der Therapie.

28. Zusammensetzung, welche eine Nukleinsäure, ein cytoplasmatisches Signalprotein oder ein chimäres Rezeptorprotein nach einem der vorhergehenden Ansprüche in Verbindung mit einem pharmazeutisch verträglichen Arzneimittelträger umfaßt.

29. Verwendung einer Nukleinsäure oder eines cytoplasmatischen Signalproteins oder eines chimären Rezeptorproteins oder einer Zusammensetzung nach einem der vorhergehenden Ansprüche bei der Herstellung eines Medikaments zur Behandlung oder Verhinderung von Erkrankungen in Menschen oder in Tieren.

30. Wirtszelle, welche eine Nukleinsäure nach einem der Ansprüche 1 bis 23 oder einen Vektor nach Anspruch 24 enthält.

31. Wirtszelle, welche ein cytoplasmatisches Signalprotein nach Anspruch 25 oder ein chimäres Rezeptorprotein nach Anspruch 26 exprimiert.

## Revendications

1. Acide nucléique codant pour un motif non naturel de transmission de signal primaire stimulateur, comprenant la séquence d'acides aminés : ou un de ses variants, qui diffère par sa séquence d'acides aminés en un nombre de positions allant jusqu'à 11 et comprend la séquence consensus X₂-L/I-X₆₋₈-Y-X₂-L/I, où X représente un acide aminé quelconque et où le chiffre en indice indique le nombre de résidus présents à cette position au sein du consensus.

2. Acide nucléique codant pour un variant de SB14^{a} selon la revendication 1, dans lequel le variant comporte au moins une variation de la séquence d'acides aminés de SB 14^{a}.

3. Acide nucléique codant pour un variant de SB 14^{a} selon la revendication 2, dans lequel le variant comporte entre 1 et 8 variations de la séquence d'acides aminés de SB14^{a}.

4. Acide nucléique codant pour un variant de SB 14^{a} selon la revendication 3, dans lequel le variant comporte entre 1 et 7 variations de la séquence d'acides aminés de SB14^{a}.

5. Acide nucléique selon l'une quelconque des revendications précédentes, dans lequel le variant est SB15^{a} qui a la séquence d'acides aminés

6. Acide nucléique selon la revendication 1, dans lequel le motif non naturel de transmission de signal primaire stimulateur est SB14^{a} qui a la séquence d'acides aminés :

7. Acide nucléique codant pour une molécule de transmission de signal cytoplasmique, qui comprend un motif non naturel de transmission de signal primaire stimulateur selon l'une quelconque des revendications précédentes et au moins un autre motif de transmission de signal primaire.

8. Acide nucléique selon la revendication 7, dans lequel ledit au moins un autre motif de transmission de signal primaire est un motif d'activation à base de tyrosine d'immunorécepteur (ITAM).

9. Acide nucléique selon la revendication 8, dans lequel ledit au moins un autre motif de transmission de signal primaire est dérivé de la chaîne ξ du TCR, FcRγ, FcRβ, CD3γ, CD3δ, CD3ε, CD5, CD22, CD79a, CD79b ou CD66d.

10. Acide nucléique selon la revendication 7, dans lequel ledit au moins un autre motif de transmission de signal primaire est un motif non naturel de transmission de signal primaire tel que défini dans l'une quelconque des revendications 1 à 6.

11. Acide nucléique selon la revendication 10, dans lequel le motif non naturel de transmission de signal primaire est SB14^{a} ou SB15^{a} comme définis respectivement dans les revendications 6 et 5.

12. Acide nucléique selon la revendication 7, dans lequel ledit au moins un autre motif de transmission de signal primaire est un motif d'inhibition à base de tyrosine d'immunorécepteur (ITIM).

13. Acide nucléique codant pour une molécule de transmission de signal cytoplasmique, qui comprend un motif non naturel de transmission de signal primaire stimulateur selon l'une quelconque des revendications 1 à 6 et au moins une séquence de transmission de signal secondaire.

14. Acide nucléique selon la revendication 13, dans lequel ladite au moins une séquence de transmission de signal secondaire est dérivée de CD28, CD 134 ou CD 154.

15. Acide nucléique selon la revendication 14, dans lequel ladite au moins une séquence de transmission de signal secondaire est ou

16. Acide nucléique codant pour une molécule de transmission de signal cytoplasmique, qui comprend un motif stimulateur non naturel selon l'une quelconque des revendications 1 à 6 et au moins un autre motif de transmission de signal primaire et au moins une séquence de transmission de signal secondaire.

17. Acide nucléique codant pour une protéine de récepteur chimère, qui comprend un domaine extracellulaire de fixation du ligand, un domaine transmembranaire et un domaine de transmission de signal cytoplasmique, le domaine de transmission de signal cytoplasmique étant codé par un acide nucléique selon l'une quelconque des revendications précédentes.

18. Acide nucléique selon la revendication 17, dans lequel le domaine extracellulaire de fixation du ligand est un domaine de fixation d'anticorps, ou un de ses fragments.

19. Acide nucléique selon la revendication 18, dans lequel le domaine extracellulaire de fixation du ligand est un fragment Fab' ou un scFv.

20. Acide nucléique selon la revendication 19, dans lequel le domaine extracellulaire de fixation du ligand est un scFv

21. Acide nucléique selon l'une quelconque des revendications 17 à 20, dans lequel le domaine transmembranaire est dérivé de la chaîne α, β ou ξ du récepteur des cellules T, CD28, CD3ε, CD45, CD4, CD5, CD8, CD9, CD16, CD22, CD33, CD37, CD64, CD80, CD86, CD134, CD137 ou CD154.

22. Acide nucléique selon la revendication 21, dans lequel le domaine transmembranaire est dérivé de CD28.

23. Acide nucléique selon l'une quelconque des revendications 17 à 20, dans lequel le domaine transmembranaire est synthétique.

24. Vecteur comprenant un acide nucléique selon l'une quelconque des revendications précédentes.

25. Protéine de transmission de signal cytoplasmique codée par un acide nucléique selon l'une quelconque des revendications 1 à 16.

26. Protéine de récepteur chimère codée par un acide nucléique selon l'une quelconque des revendications 17 à 23.

27. Acide nucléique, protéine de transmission de signal cytoplasmique ou protéine de récepteur chimère selon l'une quelconque des revendications précédentes, à utiliser en thérapie.

28. Composition comprenant un acide nucléique, une protéine de transmission de signal cytoplasmique ou une protéine de récepteur chimère selon l'une quelconque des revendications précédentes, conjointement avec un excipient pharmaceutiquement acceptable.

29. Utilisation d'un acide nucléique, ou d'une protéine de transmission de signal cytoplasmique, ou d'une protéine de récepteur chimère, ou d'une composition selon l'une quelconque des revendications précédentes, dans la fabrication d'un médicament destiné au traitement ou à la prévention d'une maladie chez les humains ou chez les animaux.

30. Cellule hôte contenant un acide nucléique selon l'une quelconque des revendications 1 à 23, ou un vecteur selon la revendication 24.

31. Cellule hôte exprimant une protéine de transmission de signal cytoplasmique selon la revendication 25 ou une protéine de récepteur chimère selon la revendication 26.
